# EUROPÄISCHE PATENTSCHRIFT

(11) **EP 2 883 603 B1**
(45) Veröffentlichungstag und Bekanntmachung des Hinweises auf die Patenterteilung: **06.07.2016**
(21) Anmeldenummer: 14189353.7
(22) Anmeldetag: 17.10.2014
(51) Int. Cl.: B01F 11/00, B01F 13/00, B01F 15/02

(54) **Vorrichtung zum Lagern und Mischen von Knochenzement**
Device for storing and mixing bone cement
Dispositif de stockage et de mélange de ciment osseux

(30) Priorität: 16.12.2013 DE 102013226118
(43) Veröffentlichungstag der Anmeldung: 17.06.2015
(73) Patentinhaber: Heraeus Medical GmbH, 61273 Wehrheim (DE)
(72) Erfinder: Dr. Vogt, Sebastian, 99092 Erfurt (DE)
(74) Vertreter: Panten, Kirsten

(56) Entgegenhaltungen:
- EP-A1- 2 823 881
- WO-A1-89/05763
- WO-A2-2007/053870
- DE-A1- 2 808 230
- US-A- 3 144 966

## Beschreibung

Die Erfindung betrifft eine Vorrichtung zur Lagerung, Vermischung und Applikation von Polymethylmethacrylat-Knochenzement und ein Verfahren zum Herstellen eines Polymethylmethacrylat-Knochenzementteigs mit einer solchen Vorrichtung.

Gegenstand der Erfindung ist somit eine Vorrichtung zur Lagerung und Vermischung von Polymethylmethacrylat-Knochenzement, der vor der Vermischung, während der Lagerung, aus einer flüssigen oder pastenförmigen ersten Komponente A und dazu separaten pulverförmigen oder pastenförmigen zweiten Komponente B besteht, sowie ein Verfahren zum Vermischen und gegebenenfalls zum Applizieren der Komponente A mit der Komponente B.

In der Medizin werden seit Jahrzehnten Polymethylmethacrylat-Knochenzemente (PMMA-Knochenzemente) zur dauerhaften mechanischen Fixierung von Totalgelenkendoprothesen eingesetzt. Diese basieren auf Pulver-Flüssigkeits-Systemen, wobei Methylmethacrylat üblicherweise als Monomer verwendet wird. Es wurden in jüngster Zeit auch Polymethylmethacrylat-Knochenzemente vorgeschlagen, die auf der Verwendung von Zementpasten beruhen (DE 10 2007 050 762 B3, DE 10 2008 030 312 A1, DE 10 2007 052 116 A1). Bei diesen Knochenzementen werden zwei Zementpasten separat in geeigneten Kartuschen gelagert. Diese enthalten jeweils neben mindestens einem Monomer und geeigneten Polymeren Bestandteile eines Redoxinitiatorsystems.

Das am häufigsten in Polymethylmethacrylat-Knochenzementen verwendete Monomer ist Methylmethacrylat. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Komponenten der Redoxinitiatorsysteme zusammenwirken. Deshalb werden die Komponenten des Redoxinitiatorsystems in den separaten Zementpasten so angeordnet, dass diese keine radikalische Polymerisation auslösen können. Die Zementpasten sind lagerstabil. Erst bei Vermischung der beiden Zementpasten zu einem Zementteig reagieren die zuvor getrennt in beiden Pasten gelagerten Komponenten des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet. Zur Vermischung der Zementpasten werden üblicherweise statische Mischer eingesetzt, die dazu an den ZweiKomponentenkartuschen angebracht werden.

Beim Auspressen der beiden Zementpasten aus den Kartuschen werden die beiden Zementpasten durch einen statischen Mischer gedrückt. Der Auspress- und der Vermischungsvorgang erfolgen dadurch gleichzeitig. Zur Vermischung der Zementpasten im statischen Mischer ist eine hohe Auspresskraft notwendig, weil der Druckabfall innerhalb des statischen Mischers an den Mischelementen sehr hoch ist. Dadurch ist es notwendig, leistungsfähige pneumatische oder mechanische Auspressvorrichtungen einzusetzen, um einen Austrag und eine Vermischung der Zementpasten zu erreichen. Diese pneumatischen oder auch mechanischen Auspressvorrichtungen sind technisch aufwendig und teuer. Kostengünstig sind dagegen die, bisher bei den auf Pulver-Flüssigkeits-Systemen beruhenden Polymethylmethacrylat-Knochenzementen üblichen, manuell betriebenen Auspresspistolen, welche sich für diese Zemente eignen, jedoch für die Auspressung und Vermischung von Knochenzementpasten unter Verwendung von statischen Mischern nicht ausreichend leistungsfähig sind.

Bei konventionellen Zweikomponentenkartuschen ist das Volumenverhältnis der Komponenten A zur Komponente B 1:1, 1:2 und 1:10. Je ungleicher die Volumina der mit statischen Mischern zu vermischenden Komponenten sind, umso schwieriger ist es eine homogen gemischte Knochenzement-Paste zu erzeugen. Deshalb sind sehr viele Mischwendeln bei größeren Volumenverhältnissen notwendig. Je größer die Anzahl der benötigten Mischwendeln ist, umso größer ist auch der Druckabfall im statischen Mischer beim Mischprozess. Es muss eine pastenförmige Komponente A vorhanden sein, die zweite Komponente B kann flüssig oder pulverförmig oder ebenfalls pastenförmig sein.

Die DE 28 08 230 A1 offenbart eine Vorrichtung entsprechend dem Oberbegriff des Anspruch 1. Sie offenbart eine Spritz-Kartusche, bei der drei Komponenten in zwei Kammern einer Kartusche und in einem Austragsrohr gelagert sind, wobei die drei Behältnisse durch eine aufreißbare beziehungsweise durchbrechbare Aluminium-Folie voneinander getrennt sind. Aus der US 3 144 966 A und der EP 2 823 881 A1 sind ähnliche Systeme bekannt, bei denen eine Folie als trennendes Element mit einem Dorn aufgestochen beziehungsweise aufgeschnitten wird, um die Behältnisse zweier Komponenten miteinander zu verbinden und so die Komponenten miteinander mischen zu können. Die WO 89/05763 A1 offenbart einen verschiebbaren Verschluss zur Trennung zweier Komponenten, wobei der Verschluss beim Verschieben Öffnungen freilegt, durch die die Komponenten gemischt werden können.

In der Klebstoff- und Dichtungsmittelindustrie hat sich seit Jahren das System Semkit^{®} bewährt. Dabei wird in einem Lagerbehälter eine Paste gelagert. In einem Rührstab ist eine zweite flüssige Komponente enthalten, die durch ein im Rührstab integriertes Ventil von der Paste getrennt ist. Bei Betätigen des Ventils läuft die Flüssigkeit in die Paste, die dann manuell gemischt werden kann.

Nachteilig ist jedoch an einem solchen System, dass das Ventilsystem nur für zähflüssige Medien geeignet ist. Das in pastenförmigen Polymethylmethacrylat-Knochenzementen übliche Monomer Methylmethacrylat kann mit diesem Ventil nicht dauerhaft von der Paste getrennt werden. Weiterhin werden bei diesem System Volumenschwankungen, die bei der axialen Mischbewegung des Rührstabes in nicht kompressiblen Pasten auftreten, dadurch ausgeglichen, dass einerseits die Kartuschen weich sind und dass anderseits die Durchführung für den Rührstab nicht vollständig dicht ist, so dass gemischte Paste austreten kann und auch Luft in geringem Umfang in die gemischte Paste gezogen werden kann. Für pastenförmige Knochenzemente sind formstabile, feste Kartuschen notwendig, weil der sehr zähe pastenförmige Polymethylmethacrylat-Knochenzement nur mit großen Auspresskräften aus Lagerbehältern ausgepresst werden kann. Weiterhin ist es für Knochenzemente nicht möglich, ein Mischsystem zu verwenden, bei dem ein unerwünschter Austritt geringer Pastenmengen auftritt und bei dem die Möglichkeit besteht, dass Luft in die Paste gezogen wird. Dadurch würde einerseits die Sauberkeit im OP beeinträchtigt und andererseits wird durch Eintrag von Luft der Zementteig mechanisch geschwächt, weil Luftblasen im ausgehärteten Zement als Rissansatzstellen wirken und damit die Festigkeit des ausgehärteten Knochenzements herabsetzen. Dadurch kann das Semkit^{®}-System nicht für pastenförmige Polymethylmethacrylat-Knochenzemente eingesetzt werden.

Die Aufgabe der Erfindung besteht also darin, die Nachteile des Stands der Technik zu überwinden. Insbesondere soll eine kostengünstige Vorrichtung zur Lagerung und Vermischung von Polymethylmethacrylat-Knochenzement entwickelt werden, mit der zumindest eine Polymethylmethacrylat-Knochenzementkomponente unter Luftausschluss gelagert werden kann, wobei nach Vermischung der Zementkomponenten ein Austragen des Zementteigs mit üblichen, preiswerten, manuell betriebenen Zementierpistolen möglich sein soll. Dabei soll die Hauptkomponente A des Polymethylmethacrylat-Knochenzements eine Zementpaste sein und die zweite Komponente kann pastenförmig sein oder bevorzugt als Pulver vorliegen. Es darf beim Vermischen nicht zum ungewollten Austritt von Zementteig und auch nicht zum Ansaugen von Luft in den Zementteig infolge von Volumenschwankungen beim Mischvorgang kommen. Die Vorrichtung soll auch geeignet sein, bei einem Volumenverhältnis der Pasten von 1:10 bis 1:30 eine sichere Vermischung der beiden Pasten zu gewährleisten, damit ein homogener Zementteig erhalten wird. Die beiden Komponenten des Knochenzementes sollen getrennt gelagert werden können und durch Betätigung einer Verschlussvorrichtung sicher zusammengeführt werden können.

Es soll mit der vorliegenden Erfindung auch erreicht werden, dass in dem Zementteig möglichst keine Rückstände durch ein Öffnen oder Aufschneiden einer Folie, wie eine Verpackung oder einer Schutzfolie verbleiben können. Die Öffnung, durch die die zweite Komponente in die Hauptkomponente geleitet wird, soll eine reproduzierbare Querschnittsfläche aufweisen und sich möglichst nicht beim Mischen der Komponenten verändern. Zudem soll die Öffnung des Austragsrohrs zum Behälter, in dem die Komponenten gemischt werden, immer eine vorherbestimmte Querschnittsfläche aufweisen. Es soll möglichst nicht zu einer Beeinträchtigung dieser Querschnitte kommen können.

Eine weitere Aufgabe der Erfindung besteht darin, dass nach erfolgter Mischung der Zementkomponenten und erfolgter Öffnung des Verschlusses das Austragsrohr der Vorrichtung für den Zementteig sicher durchgängig sein muss, wobei die Öffnung des Austragsrohrs gegen ein Blockieren durch den geöffneten Verschluss während des Auspressens des Zementteigs gesichert sein muss. Weiterhin soll ein Verfahren zum Vermischen von pastenförmigen Polymethylmethacrylat-Knochenzementen unter Verwendung der zu entwickelnden Vorrichtung bereitgestellt werden.

Die Aufgaben der Erfindung werden gelöst durch eine Vorrichtung entsprechend Anspruch 1.

Die erste Komponente ist vorzugsweise luftfrei. Die zweite Komponente ist bevorzugt pulverförmig oder pastenförmig, besonders bevorzugt eine selbststerilisierende Paste. Selbststerilisierende Pasten können beispielsweise Wasserstoffperoxid enthalten. Bevorzugt ist in dem zweiten Behälter eine selbststerilisierende Paste enthalten, wie sie in der EP 2 596 812 A1 beschrieben ist.

Grundsätzlich reicht es zur Realisierung des Erfindungsgedankens aus, wenn das Austragsrohr bezogen auf die Wirkverbindung gegenüberliegend zum Austragskolben angeordnet ist. Eine exakte geometrische Gegenüberstellung ist nicht erforderlich.

Ein Volumenausgleichselement kann bevorzugt durch einen oder zwei axial im zylindrischen Innenraum des ersten Behälters bewegliche Zylinder realisiert sein. Alternativ oder zusätzlich kann ein Volumenausgleichselement auch durch eine flexible verformbare Haut oder Membran gebildet sein.

Es wird vorgeschlagen, dass der Verschluss eine Kappe ist, die auf dem ins Innere weisende ersten Ende des Austragsrohrs steckt oder angeordnet ist, oder der Verschluss ein Stopfen ist, der in dem ins Innere weisende ersten Ende des Austragsrohrs steckt oder angeordnet ist.

Diese Ausführungen sind besonders einfach zu realisieren und dadurch kostengünstig im Aufbau.

Ein Stopfen wird erfindungsgemäß bevorzugt, da dieser leichter mit einem erfindungsgemäß vorgeschlagenen Sicherungsring kombiniert werden kann, der über das erste Ende des Austragsrohrs schiebbarist.

Mit einer Weiterbildung der Erfindung wird auch vorgeschlagen, dass die verformbare Verbindung ein Steg ist, der gebogen ist, wenn der Verschluss das Austragsrohr verschließt, wobei vorzugsweise der Steg und der Verschluss einteilig ausgeführt sind.

Der Steg stellt eine besonders einfach und kostengünstig zu realisierende Verbindung dar. Über einen solchen gebogenen Steg kann die Federkraft eines elastischen Stegs genutzt werden, um den Verschluss von der inneren Öffnung des Austragsrohrs (am ersten Ende des Austragsrohrs) weg zu bewegen.

Des Weiteren kann vorgesehen sein, dass die verformbare Verbindung gespannt ist und eine Federkraft auf den Verschluss bewirkt, die den Verschluss von der Öffnung des ersten Endes des Austragsrohrs weg bewegt, wenn der Verschluss von dem ersten Ende des Austragsrohrs gelöst ist. Insbesondere, wenn der Verschluss durch die axiale Bewegung des Kerns von dem ersten Ende des Austragsrohrs gelöst ist.

Dadurch kann gefördert werden oder bei geeignetem Aufbau sogar sichergestellt werden, dass der geöffnete Verschluss nicht die Öffnung ins Austragsrohr (am ersten Ende des Austragsrohrs) beeinträchtigt. Die Verbindung sollte dazu eine ausreichende Elastizität mit einem geeigneten Elastizitätsmodul aufweisen. Hierfür sind beispielsweise ca. 1 mm dicke und 3 mm breite Kunststoffstege aus Polyethylen oder einem anderen nicht zu harten Kunststoff beispielsweise einem üblichen Thermoplast geeignet.

Mit der Erfindung wird ferner vorgeschlagen, dass an dem ins Innere weisenden ersten Ende des Austragsrohrs ein Sicherungselement angeordnet ist und mit dem Sicherungselement der geöffnete Verschluss arretierbar oder derart in seiner Beweglichkeit gegenüber der Öffnung des ersten Endes des Austragsrohrs einzuschränken ist, dass die Öffnung des ersten Endes des Austragsrohrs durch den Verschluss nicht mehr verschließbar ist oder zu verkleinern ist.

Dass die Öffnung nicht mehr mit dem Verschluss zu verkleinern ist, bedeutet, dass sich der Verschluss nicht derart vor oder an die Öffnung legen kann, dass die Strömung in das Austragsrohr unmittelbar durch den Verschluss behindert wird. Der freie Querschnitt der Öffnung des Austragsrohrs am ersten Ende wird also nicht durch den mit dem Sicherungselement gesicherten Verschluss verkleinert.

Erfindungsgemäß bevorzugt ist die Mischeinrichtung zwischen dem Sicherungselement und der Durchführung am Austragsrohr angeordnet.

Mit diesem Aufbau kann sichergestellt werden, dass der geöffnete Verschluss nicht die Öffnung ins Austragsrohr beeinträchtigt.

Gemäß einer bevorzugten Weiterbildung kann vorgesehen sein, dass an dem Sicherungselement eine zum Außenumfang des Austragsrohrs beabstandete Strebe angeordnet ist, die den Steg des Verschlusses in Richtung des zweiten Endes des Austragsrohrs biegt, wenn das Sicherungselement bedient, insbesondere bewegt wird. Besonders bevorzugt wird das Sicherungselement weiter auf das erste Ende des Austragsrohrs aufgeschoben und dadurch der Steg mit der Strebe umgebogen und die Strebe schiebt sich dabei über den Steg.

Bei erfindungsgemäßen Vorrichtungen mit Sicherungselement ist vorgesehen, dass das Sicherungselement ein Sicherungsring ist, der auf das erste Ende des Austragsrohrs aufgesteckt ist, der in seiner Ausgangslage über das erste Ende hinaus ragt und der weiter auf das erste Ende des Austragsrohrs aufschiebbar ist, wobei das Aufschieben des Sicherungsrings den Verschluss sichert, in dem durch die neue Lage des Sicherungsrings die Deformierbarkeit der verformbaren Verbindung eingeschränkt ist.

Bevorzugt kann vorgesehen sein, dass der Sicherungsring eine Ausnehmung zur Aufnahme des Stegs aufweist, mit dem zumindest ein vollständiges Biegen des Stegs zur Öffnung in das Austragsrohr zu verhindern ist. Ebenso kann bevorzugt vorgesehen sein, dass an dem Innenumfang des Sicherungsrings zumindest eine Rastung vorgesehen ist, die in zumindest eine Gegenrastung am Außenumfang des Austragsrohrs greift, wobei die Rastung ein Lösen des Sicherungsrings von dem Austragsrohr verhindert. Besonders bevorzugt sind zwei Rastungsstufen vorgesehen, wobei die erste Rastungsstufe ein Lösen des Sicherungsrings von dem Austragsrohr verhindert und die zweite Rastungsstufe ein Lösen der Sicherung des Verschlusses verhindert.

Dies sind besonders einfache und kostengünstig zu realisierende Möglichkeiten das Sicherungselement aufzubauen.

Mit einer besonders bevorzugten Ausführungsform der Erfindung kann auch vorgesehen sein, dass der Sicherungsring eine nicht durchgehende, in Richtung des Austragskolbens weisende axiale Ausnehmung aufweist und daran in axialer Richtung in Richtung der Vorderseite der Vorrichtung anschließend eine radiale Ausnehmung aufweist, so dass die axiale Ausnehmung von einer Strebe mit radialem Abstand zum Außenumfang des Austragsrohrs überbrückt ist.

Bei erfindungsgemäßen Vorrichtungen kann vorgesehen sein, dass der Austragskolben gegen den ersten Behälter arretierbar ist oder arretiert ist, bevorzugt an dem dem Austragsrohr gegenüber liegenden Ende des ersten Behälters.

Mit der Arretierung kann der Austragskolben beim Entgasen und Sterilisieren des ersten Behälters gehalten werden.

Mit einer bevorzugten Ausgestaltung der Erfindung wird ferner vorgeschlagen, dass das Austragsrohr über eine gasdichte Durchführung durch eine Begrenzung des ersten Behälters hindurch axial beweglich ist, so dass die Mischeinrichtung durch eine Bewegung des Austragsrohrs zum Durchmischen des Inhalts des ersten Behälters bewegbar ist, wobei bevorzugt das Austragsrohr axial drehbar gelagert ist.

Hierdurch wird der Eintrag von Luft vermieden. Zudem bleibt so der Inhalt der Behälter steril.

Die Mischeinrichtung kann über eine Mehrzahl von Mischflügeln realisiert werden. Es ist auch vorstellbar, dass die Mischeinrichtung über das Austragsrohr bedienbar ist und zusätzlich über einen separaten oder integrierten Magnetrührkern verfügt.

Hierdurch kann auf einfache Weise eine manuelle Durchmischung des Inhalts erfolgen.

Es kann des Weiteren vorgesehen sein, dass die Austragsöffnung am zweiten Ende des Austragsrohrs verschlossen ist und vor der Applikation des gemischten Knochenzements zu öffnen ist.

Bevorzugt kann vorgesehen sein, dass ein Griffstück zum manuellen Bewegen der Mischeinrichtung am Austragsrohr vorgesehen sein.

Bevorzugt kann vorgesehen sein, dass der erste Behälter einen zylindrischen Innenraum aufweist und der Austragskolben in dem Innenraum des ersten Behälters eine Passform entsprechend der Grundfläche des zylindrischen Innenraums aufweist.

Unter einem zylindrischen Innenraum ist geometrisch ein allgemeiner Zylinder mit einer beliebigen Grundfläche zu verstehen, also nicht nur einer, mit einer kreisförmigen Grundfläche. Der Innenraum kann also ein gerader Zylinder mit beliebiger Grundfläche sein, das heißt auch mit nicht kreisförmiger oder runder Grundfläche. Erfindungsgemäß wird aber ein zylindrischer Innenraum mit kreisrunder Grundfläche bevorzugt. Diese Geometrie macht alle Bereiche des ersten Behälters für die Mischeinrichtung besonders gut erreichbar. Der Austragskolben ist dann ebenfalls zylindrisch und liegt vorzugsweise über eine Dichtung an den Wandungen des zylindrischen Innenraums des ersten Behälters an. Besonders bevorzugt ist an der dem Innenraum zugewandten Seite des Austragskolbens ein Abstreifer angeordnet, mit dem verhindert wird, dass die gemischte Knochenzementpaste beim Vortreiben des Austragskolbens am Austragskolben vorbei gedrückt wird und auf der Rückseite der Vorrichtung austreten kann. Die Mischeinrichtung hat, bei der bevorzugten kreisrunden Zylindergeometrie des ersten Behälters, Mischflügel, die gleich oder bevorzugt etwas kleiner als der Innendurchmesser des zylindrischen Innenraums sind.

Die zylindrische Geometrie mit kreisrunder Grundfläche ist die einfachste zum Zweck des Aufbaus der Vorrichtung. Es ist besonders bevorzugt, wenn auch die Außenfläche des ersten Behälters entsprechend zylindrisch ist und zu wenigstens 90% die Wandung des Behälters über eine gleichmäßige Dicke verfügt. Dann kann der erste Behälter seitlich als einfaches Rohr aufgebaut werden.

Gemäß einer bevorzugten Ausführung der Erfindung kann vorgesehen sein, dass zumindest ein Volumenausgleichselement axial beweglich in oder an dem ersten Behälter angeordnet ist, wobei bevorzugt das zumindest eine Volumenausgleichselement die gasdichte Durchführung umfasst, durch die das Austragsrohr zum Bedienen der Mischeinrichtung hindurchgeführt ist.

Der Aufbau hierzu ist einfach umsetzbar und dadurch kostengünstig zu realisieren.

Gemäß einer weiteren Vereinfachung der Erfindung kann vorgesehen sein, dass ein Volumenausgleichselement durch den Austragskolben realisiert ist und die Bewegung des Austragskolbens aus dem ersten Behälter heraus durch ein Begrenzungselement begrenzt ist, wobei das Begrenzungselement bevorzugt eine Rastung ist, die in eine Gegenrastung am Austragskolben greift.

Alternativ kann das Begrenzungselement auch durch eine Überwurfmutter gebildet sein, mit der der Austragskolben fixierbar ist. Auf der gegenüberliegenden Seite kann eine Überwurfmutter mit einer Klemmvorrichtung vorgesehen sein, mit der das Austragsrohr nach Art eines Bohrfutters gegen den Behälter fixierbar ist.

Die Verwendung des Austragskolbens als Volumenausgleichselement ist deshalb besonders einfach, da der Austragskolben ohnehin beweglich innerhalb des ersten Behälters beziehungsweise genauer innerhalb der Wandungen des bevorzugt zylindrischen Kunststoffkörpers sein soll. Damit kann dieser auch für den Volumenausgleich verwendet werden und es ist kein weiteres bewegliches Volumenausgleichselement mehr notwendig.

Bevorzugt kann vorgesehen sein, dass ein Volumenausgleichselement gegenüber dem Austragskolben in dem ersten Behälter als beweglich gelagertes Volumenausgleichselement angeordnet ist, wobei bevorzugt das Volumenausgleichselement die gasdichte Durchführung für das Austragsrohr umfasst.

Es können auch beide genannten Volumenausgleichselemente gleichzeitig verwirklicht sein durch ein erstes Volumenausgleichselement, das durch den Austragskolben realisiert ist, und durch ein zweites Volumenausgleichselement, das gegenüber dem Austragskolben in dem ersten Behälter als beweglich gelagertes zylindrisches Volumenausgleichselement angeordnet ist. Zusätzlich könnte theoretisch auch eine verformbare Membran als drittes Volumenausgleichselement vorgesehen sein, die ebenfalls eine Volumenänderung des ersten Behälters durch Bedienen der Mischeinrichtung und/oder durch Einfüllen der zweiten Komponente aus dem zweiten Behälter in den ersten Behälter aufnehmen kann. Zur Vereinfachung des Aufbaus ist jedoch die Beschränkung auf ein einziges Volumenausgleichselement bevorzugt.

Das gegenüber zum Austragskolben angeordnete Volumenausgleichselement hat den Vorteil, dass es bezüglich seiner Beweglichkeit voll auf die Anforderungen eines Volumenausgleichselements abgestimmt werden kann.

Ferner kann vorgesehen sein, dass der Kern als Verschlusselement der Austragöffnung des Austragsrohrs vorgesehen ist, wobei die Austragöffnung der ersten Öffnung gegenüberliegend angeordnet ist, wobei der Kern aus dem Austragsrohr herausnehmbar ist.

Durch den Kern kann der gesamte Inhalt aus dem zweiten Behälter herausgepresst werden.

Dabei kann vorgesehen sein, dass der Kern an der dem ersten Behälter zugewandten Seite einen Abstreifer aufweist, der beim Herausziehen des Kerns Pulver oder Zementteig an der Innenseite des Austragsrohrs abstreift.

Hierdurch kann vermieden werden, dass Rückstände im Austragsrohr zurückbleiben, die beim Austragen gelöst werden und die dadurch die Eigenschaften und die Homogenität des Knochenzements beeinflussen könnten. Dadurch kann sichergestellt werden, dass der frühzeitig ausgetragene Knochenzement die gleichen Eigenschaften hat wie der später ausgetragene.

Des Weiteren kann vorgesehen sein, dass zumindest ein Volumenausgleichselement über eine elastische Feder gegen den ersten Behälter beweglich gelagert ist, wobei die Feder das Volumenausgleichselement in den Innenraum des ersten Behälters drückt.

Die Feder unterstützt die Rückbewegung des Volumenausgleichselements. Dadurch wird die durch die ebenfalls treibenden hydraulischen Kräfte durch den Knochenzementteig beziehungsweise dessen Komponenten verursachten und übertragenen Kräfte auf die Dichtungen und die beweglichen Teile der Vorrichtung reduziert, so dass ein kostengünstigerer Aufbau mit weniger Materialstärke und geringeren Anpressdrucken realisiert werden kann.

Gemäß einer bevorzugten Weiterbildung der Erfindung kann auch vorgesehen sein, dass die Seite des ersten Behälters mit dem Austragsrohr, beziehungsweise durch die das Austragsrohr durchgeführt ist, von einer Verschlusskappe verschlossen ist, die eine Durchführung für das Austragsrohr aufweist, die ein Volumenausgleichselement abdeckt und die Verschlusskappe zumindest eine Öffnung zum Ermöglichen eines Druckausgleichs zwischen der Umgebung und dem Zwischenraum zwischen der Verschlusskappe und dem abgedeckten Volumenausgleichselement aufweist, wobei bevorzugt eine elastische Spiralfeder zum Drücken des Volumenausgleichselements in den Innenraum des ersten Behälters zwischen der Verschlusskappe und dem abgedeckten Volumenausgleichselement angeordnet ist, wobei besonders bevorzugt die Spiralfeder um das Austragsrohr herum angeordnet ist.

Durch diese Maßnahme kann eine Beeinträchtigung der Beweglichkeit durch äußere Einflüsse (wie beispielsweise Druck oder Blockade des Volumenausgleichselements) vermieden werden.

Auch kann vorgesehen sein, dass das Volumenausgleichselement mit einem lösbaren Arretierungsmittel arretiert ist.

Hierdurch kann eine ungewollte Bewegung des Volumenausgleichselements beim Befüllen oder Sterilisieren des ersten Behälters vermieden werden.

Die der Erfindung zugrundeliegenden Aufgaben werden auch gelöst durch ein Verfahren zum Herstellen eines Polymethylmethacrylat-Knochenzements entsprechend Anspruch 17.

Der Kern dichtet die Vorrichtung nach außen ab.

Dabei kann vorgesehen sein, dass nach Schritt B) oder nach Schritt C) der Kern aus dem Austragsrohr entfernt wird und danach ein Schritt D) erfolgt, bei dem der gemischte Knochenzement durch Vortreiben des Austragskolbens in dem ersten Behälter appliziert wird.

Dies erleichtert die Applikation, da der Kern nicht durch den Vortrieb des Austragskolbens herausgestoßen werden muss. Die Applikation entspricht der finalen Anwendung der mobilen und handhabbaren Vorrichtung.

Es ist vorgesehen, dass beim Mischen der beiden Komponenten in Schritt C) das ins Innere des Behälters weisende erste Ende des Austragsrohrs bis auf den Austragkolben gedrückt wird, wobei durch den Druck ein Sicherungselement, das an diesem ersten Ende angeordnet ist, bedient wird und mit der Bedienung des Sicherungselements der geöffneten Verschluss arretiert wird oder derart in seiner Beweglichkeit gegenüber der Öffnung des ersten Endes des Austragsrohrs eingeschränkt wird, dass die Öffnung des ersten Endes des Austragsrohrs durch den Verschluss nicht mehr verschlossen werden kann oder verkleinert werden kann.

Dadurch kann sichergestellt werden, dass der geöffnete Verschluss nicht die Öffnung und den Fluss des Zementteigs ins Austragsrohr beeinträchtigt.

Des Weiteren kann vorgesehen sein, dass die Mischeinrichtung mit dem Austragsrohr verbunden ist und der Inhalt des ersten Behälters gemischt wird, indem die Mischeinrichtung durch hineinbewegen und herausbewegen des Austragsrohrs in den ersten Behälter in dem ersten Behälter bewegt wird, wobei bevorzugt zusätzlich die Mischeinrichtung durch Drehen des Austragsrohrs im ersten Behälter gedreht wird.

Hierdurch ist das Verfahren besonders einfach durchzuführen, da mit dem Austragsrohr nur ein bewegliches Element bedient wird, so dass die Wahrscheinlichkeit für eine Fehlbedienung reduziert wird. Zudem ist eine einfache Durchmischung der Komponenten auch unter widrigen Bedingungen außerhalb eines ordentlichen OP-Saals leicht möglich.

Ferner kann vorgesehen sein, dass vor Schritt A) der erste Behälter mit einer Komponente des Knochenzements gefüllt wird und das Innere des ersten Behälters zuvor entgast und sterilisiert wird, wobei dazu bevorzugt der Austragskolben und/oder das Volumenausgleichselement arretiert wird.

Hierdurch wird sichergestellt, dass der Inhalt steril ist. Dadurch können Infektionen beim Patienten vermieden werden.

Ferner kann bei erfindungsgemäßen Verfahren vorgesehen sein, dass nach erfolgter Vermischung das Austragsrohr in Richtung aus dem ersten Behälter heraus bewegt wird, so dass die Mischeinrichtung an der Innenseite des Volumenausgleichselements oder der vorderen Innenseite des Behälters anliegt.

Hierdurch kann die gesamte Kraft des Austragskolbens zum Austreiben des Zementgemischs verwendet werden.

Schließlich kann auch vorgesehen sein, dass bei dem Durchführen des Verfahrens, die Volumenänderungen in dem Behälter durch das Volumenausgleichselement ausgeglichen werden.

Der Erfindung liegt die überraschende Erkenntnis zugrunde, dass dadurch, dass der Verschluss über eine Verbindung mit dem Austragsrohr verbunden ist, eine unkontrollierte Bewegung und Positionierung des Verschlusses im Behälter verhindert werden kann, so dass ein ungewollter Versschluss des Austragsrohrs oder auch eine ungewollte Verringerung oder Beeinträchtigung des Querschnitts des Austragsrohrs ausgeschlossen werden kann. Zudem kann ein solcher Verschluss als kompakter Volumenkörper in Form einer Kappe oder eines Pfropfens gestaltet werden, so dass auszuschließen ist, dass sich von dem Verschluss Teile oder Partikel lösen, die den Knochenzement verunreinigen könnten.

Die Verwendung des Austragsrohrs als zweiten Behälter ist platzsparend und spart auch ein zusätzliches bewegliches Teil im Inneren des ersten Behälters. Bei einem erfindungsgemäßen Aufbau reicht eine einzige Durchführung aus, mit der sowohl der zweite Behälter gegen den ersten Behälter geöffnet werden kann, als auch die Mischeinrichtung bedient werden kann und auch der Zementteig appliziert werden kann.

Ferner liegt der Erfindung auch die überraschende Erkenntnis zugrunde, dass es durch die Verwendung zumindest eines beweglichen Volumenausgleichselements gelingt, eine gasdichte Vorrichtung zum Mischen von PMMA-Knochenzement bereitzustellen, bei der auch kein Knochenzement durch die beim Mischen auftretenden Volumenänderungen aus der Vorrichtung austreten kann. Hierdurch wird es möglich, einen sehr einfachen und kostengünstigen Aufbau zum Mischen von Knochenzement zu realisieren, bei dem keine Gefahr durch Kontamination der Umgebung mit dem Knochenzement besteht und bei dem auch keine Gefahr besteht, dass beim Mischen Luft oder Gas in den Knochenzement eingemischt wird, der nach dem Aushärten zu einer Schwächung des Knochenzements führt.

Durch die Nutzung des Austragsrohrs als Betätigungseinrichtung für die Mischeinrichtung sowie durch die Nutzung des Austragsrohrs als Behälter für die zweite Komponente des Knochenzements, kann auf zusätzliche Bauteile verzichtet werden. Hierdurch wird eine weitere Kostenreduktion erreicht.

Es ist erfindungswesentlich, dass in dem ersten Behälter eine Paste oder Flüssigkeit als erste Komponente enthalten ist. Ferner dürfen keine Luft und keine Gaseinschlüsse in der Paste oder der Flüssigkeit enthalten sein und es darf auch keine Gasphase in dem ersten Behälter überstehen. Jeder Gaseinschluss kann zu einer Verminderung der Qualität des erzeugten Knochenzements führen. Durch die inkompressible Paste und das Fehlen von kompressiblen Gasvolumina ist der Inhalt des ersten Behälters dadurch völlig inkompressibel. Die Durchmischung durch Einschieben und Herausziehen des Austragsrohrs, an dem die Mischeinrichtung befestigt ist, ist dann erfindungsgemäß nur mit dem Volumenausgleichselement möglich. Da der erste Behälter über starre Wandungen verfügt, könnte ansonsten kein Einschieben oder Herausziehen des Austragsrohrs erfolgen.

Die Grundidee der Erfindung basiert somit darauf, im Gegensatz zu der bei pastenförmigen Zwei-Komponenten-Polymethylmethacrylat-Knochenzementen üblichen zeitgleichen Kombination des Auspress- und des Vermischungsvorgangs unter Verwendung von statischen Mischern, den Auspress- und Vermischungsvorgang zeitlich und räumlich zu trennen. Das bedeutet, dass erst die flüssigen, pastenförmigen und/oder pulverförmigen Komponenten miteinander vermischt werden und erst danach der gebildete Zementteig ausgepresst wird. Die Vermischung erfolgt dabei durch einen manuell zu betätigenden Mischer. Dadurch ist die zum Austragen erforderliche Auspresskraft niedrig und manuell betriebene, kostengünstige Zementierpistolen können zum Auspressen des Zementteigs verwendet werden. Weiterhin ist es dadurch möglich, auch bei einem Volumenverhältnis der Pasten von 1:10 bis 1:30 eine gleichmäßige Vermischung der Pasten zu erreichen. Wesentlich für die Erfindung ist die Anordnung eines beweglichen Volumenausgleichselements mit dem Volumenschwankungen während des Mischprozesses ausgeglichen werden können, ohne das Zementteig austritt und ohne das Luft in den Zementteig gezogen wird.

Eine besonders bevorzugte Ausführungsform der Erfindung kann vorsehen, dass der Verschluss durch einen Stopfen oder eine Kappe als Verschluss gebildet wird, der oder die über einen kurzen beweglichen Steg mit dem Austragsrohr verbunden ist. Der Steg darf dabei nur eine solche Länge besitzen, dass nach halbkreisförmiger Biegung des Steges der Stopfen oder die Kappe gerade in das Austragsrohr eingesteckt beziehungsweise auf das Austragsrohr aufgesetzt werden kann. Nach Öffnung des Verschlusses durch Herausdrücken des Stopfens oder der Kappe durch den Kern im Austragsrohr hängt der Verschluss an dem kurzen, beweglichen Steg. Am Ende des Austragsrohrs befindet sich ein axial auf dem Austragsrohr verschiebbarer Sicherungsring, der eine Aussparung zur Aufnahme des Stegs aufweist. Im ungeöffneten Zustand ist der Sicherungsring nur teilweise über das Ende des Austragsrohrs geschoben. Nach Herauspressung des Stopfens oder nach Abdrücken der Kappe wird bei der ersten Mischbewegung, bei der das Austragsrohr auf den Boden des Austragskolbens drückt, der Sicherungsring axial in Richtung der Austragsöffnung des Austragsrohrs geschoben, wobei die Austragsöffnung der ersten Öffnung gegenüberliegt. Dabei wird der Steg in der Aufnahme des Sicherungsrings gegen das Austragsrohr gepresst. Dadurch ist nur noch ein Teil des Stegs beweglich. Das bedeutet, dass nach halbkreisförmiger Biegung des beweglichen Teils des Stegs die Länge des Stegs nicht ausreicht, damit der Stopfen oder die Kappe vor der Öffnung des Austragsrohrs liegen kann. Eine Blockierung der Öffnung des Austragrohrs während des Auspressens des Zementteigs ist dadurch vollständig ausgeschlossen. Durch Anordnung eines Rastelements, das bei axialer Verschiebung wirksam wird, kann ein Zurückschieben des Sicherungselements in den ungesicherten Zustand ausgeschlossen werden. Vorteilhaft kann bei dieser Ausführung auch vorgesehen sein, dass der Sicherungsring eine erste Rastung besitzt, die im ungesicherten Zustand den Sicherungsring auf dem Austragsrohr fixiert, damit dieser im ungesicherten Zustand während der Lagerung beziehungsweise während des Transports der Vorrichtung nicht vom Austragsrohr abfallen kann.

Eine beispielhafte und besonders bevorzugte Vorrichtung zur Lagerung, Vermischung und Applikation von Polymethylmethacrylat-Knochenzement ist zusammengesetzt aus
a) einem zylinderförmigen Lagerbehälter mit einem ersten Raum für eine erste pastenförmige Komponente,
b) einem verschiebbaren Austragskolben, der an einem Ende des zylinderförmigen Lagerbehälters arretiert werden kann,
c) einem Austragsrohr,
d) einem axial im Lagerbehälter beweglichen Volumenausgleichselement, das eine Durchführung für ein axial verschiebbares Austragsrohr besitzt,
e) einem ersten Verschluss des Lagerbehälters an dem zum Austragskolben gegenüber liegenden Ende des Lagerbehälters,
f) einer Durchführung des ersten Verschlusses für das axial zum Lagerbehälter verschiebbare Austragsrohr,
g) eine Mischeinrichtung, die am Ende des Austragsrohrs angebracht ist, welches in dem Raum zwischen dem Volumenausgleichselement und dem Austragskolben angeordnet ist,
h) einem in dem Austragsrohr axial beweglich angeordneten Kern,
i) einem durch axiale Bewegung des Kerns in Richtung Austragskolben zu öffnenden zweiten Verschluss des Austragsrohrs, wobei der zweite Verschluss an dem Ende des Austragsrohrs angeordnet ist, an dem sich die Mischeinrichtung befindet, wobei der zweite Verschluss über ein biegbares Verbindungsmittel mit dem Austragsrohr verbunden ist,
j) einem axial auf dem Austragsrohr verschiebbaren Sicherungsring, der mindestens eine Aufnahme für das Verbindungsmittel des zweiten Verschlusses enthält, und
k) einem zweiten Raum für eine zweite Komponente des Knochenzements, der vom Austragsrohr, vom zweiten Verschluss des Austragsrohrs und dem in Richtung des Inneren zeigenden Ende des beweglichen Kerns gebildet wird.

Die Vorrichtung besteht bevorzugt im Wesentlichen aus in der Medizin üblichen thermoplastischen Kunstoffen, wie Polypropylen, Polyethylen, Polyamid oder sonstigen medizinisch geeigneten Kunststoffen.

Das Austragsrohr mit der daran angeordnetem Mischeinrichtung und der Kern basieren auf einem im EP 2 072 114 B1 beschriebenen Zementiersystem, dass unter der Bezeichnung Palamix von der Firma Heraeus Medical GmbH (Wehrheim) hergestellt und vertrieben wird.

Die pastenförmige erste Komponente A soll luftfrei im Lagerbehälter (ersten Behälter) gelagert werden. Das bedeutet, es darf keine Gasphase über der pastenförmigen Komponente A stehen. Dadurch gibt es kein kompressibles Medium, das beim Eintrag der Komponente B und bei dem durch den Mischvorgang durch Eintauchen und Herausziehen des Mischers auftretenden Volumenzunahmen und Volumenabnahmen kompensieren kann. Es muss unbedingt vermieden werden, dass beim Mischprozess Paste heraustritt beziehungsweise, dass Luft in die Paste gesaugt wird.

Wesentlich für die Erfindung ist daher, dass ein Volumenausgleichselement realisiert wird, das bevorzugt durch einen axial im Lagerbehälter beweglichen Volumenausgleichskolben gebildet wird, welcher eine Ausnehmung zur Durchführung des Austragsrohrs besitzt. Das als beweglicher Kolben ausgebildete Volumenausgleichselement kann durch axiale Bewegung in Richtung des Verschlusses die Volumenzunahme zwischen dem Austragskolben und dem Volumenausgleichselement durch Eintrag der Komponente B in die Paste A und auch den Volumeneintrag durch Bewegung des Austragsrohrs in Richtung des Austragskolbens ausgleichen. Bei der Bewegung des Austragsrohrs in Richtung des Verschlusses wird das sich verringernde Volumen zwischen dem Austragskolben und dem Ausgleichselement durch Bewegung des Volumenausgleichselements in Richtung des Austragskolbens kompensiert. Dadurch ist ein Mischen möglich, ohne dass Luft bei der Volumenzunahme eintritt und ohne dass Zementpaste während der temporären Volumenzunahme infolge der dabei auftretenden Druckerhöhung austreten kann.

Es ist erfindungsgemäß, dass das Volumenausgleichselement durch einen axial im Lagerbehälter beweglichen Kolben gebildet wird, welcher eine Ausnehmung zur Durchführung des Austragsrohrs besitzt.

Es ist möglich das Volumenausgleichselement aus einem Kolben zu bilden, der ein Entlüftungsventil aufweist.

Vorteilhaft ist es, wenn das Volumenausgleichselement mit einer Feder, die sich an dem vorderen Verschluss der Kartusche (ein Deckel des Grundkörpers) abstützt, vom ersten Verschluss beabstandet wird, wobei eine Spiralfeder, die um das Austragsrohr geführt ist, bevorzugt ist.

Weiterhin ist es vorteilhaft, wenn das Volumenausgleichelement durch ein Arretierungsmittel vor dem Mischprozess gegen Verschiebung gesichert ist, wobei mindestens ein axial zum Austragsrohr angeordneter Stift (Sicherungsstift) bevorzugt ist, der durch eine Durchführung des ersten Verschlusses (des Deckels der Kartusche) nach außen ragt und mit einer Rastung am ersten Verschluss arretiert ist oder arretierbar ist. Durch kann der medizinische Anwender vor dem Mischprozess das Volumenausgleichselement durch Entfernung des Sicherungsstifts lösen.

Es kann also erfindungsgemäß vorgesehen sein, dass das Volumenausgleichselement während der Befüllung des Lagerbehälters durch eine Splint, einen Stift oder eine andere Arretierungsmittel gegen Verschieben gesichert ist, der nach der Befüllung oder vor der Vermischung der pastenförmigen ersten Komponente A mit der zweiten Komponente B entfernt wird.

Besonders vorteilhaft kann bei dieser Variante vorgesehen sein, dass der (zweite) Verschluss des ersten Endes des Austragsrohrs als ein Stopfen ausgebildet ist, der über einen biegbaren Steg mit dem Austragsrohr verbunden ist, wobei der Stopfen im verschlossenen Zustand in das Austragsrohr eingesteckt ist. Der Steg hat nur eine solche Länge, dass nach halbkreisförmiger Biegung des Stegs der Stopfen gerade in das Austragsrohr eingesteckt werden kann.

Der Sicherungsring ragt über den Rand des Austragsrohr heraus, wobei sich die Aufnahme des Stegs unter der Verbindungsstelle des Stegs mit dem Austragsrohr befindet oder dass sich teilweise der Steg in der Aufnahme befindet.

Nach Öffnung des Verschlusses und des Aufsetzens des Austragsrohrs auf die Oberseite des Austragskolbens wird der Sicherungsring axial in Richtung Kartuschenverschluss (erster Verschluss) verschoben, wobei der Steg durch die Aufnahme des Sicherungsrings in Richtung des Austragsrohr gepresst wird und die Unterseite des Sicherungsrings mit der Unterkante des Austragsrohrs abschließt. Dadurch ist nur noch ein Teil des Stegs verbiegbar oder verformbar. Der Radius eines derart gebogenen Halbkreises des Stegs ist kleiner als im ungesicherten Ausgangszustand. Das bedeutet, dass es geometrisch unmöglich ist, dass sich der Stopfen vor die Öffnung des Austragsrohrs legen kann. Dadurch wird ein Blockieren der Öffnung wirksam vermieden.

In einer alternativen Ausführungsform der Erfindung wird das Volumenausgleichselement durch eine axial deformierbare Membran gebildet, welche eine Ausnehmung zur Durchführung des Austragsrohrs besitzt.

Für die Funktion des Volumenausgleichselements ist es wesentlich, dass das Volumenausgleichselement mit einer Feder, die sich am ersten Verschluss abstützt, vom ersten Verschluss beabstandet wird, wobei hierzu eine Spiralfeder bevorzugt wird, die um das Austragsrohr geführt ist. Die Feder dient dazu, das Volumenausgleichselement nach einer temporären Volumenzunahme und damit einer Bewegung des Volumenausgleichselements in Richtung Verschluss wieder in die Ausgangslage zurück zu führen. Das bedeutet, dass kein Unterdruck im ersten Behälter notwendig ist, damit die Ausgangsposition des Volumenausgleichselements wieder erreicht wird.

Eine erfindungsgemäße Vorrichtung kann also bevorzugt eine Zementkartusche zum Lagern, Mischen und Applizieren eines Zweikomponenten-Zements sein.

Die Erfindung wird auch durch ein Verfahren entsprechend Anspruch 17 zum Vermischen von Polymethylmethacrylat-Knochenzement mit der erfindungsgemäßen Vorrichtung realisiert. In der erfindungsgemäßen Vorrichtung befindet sich eine Zementpaste A im ersten Behälter und eine durch die Membran separierte Komponente B im zweiten Behälter.

Das Verfahren kann beispielsweise dadurch realisiert werden, dass
a) das der Kern im Austragsrohr in Richtung des Stopfens bewegt wird, wodurch die zweite Komponente B den Stopfen aus dem Austragrohr herausstößt und der zweite Behälter geöffnet wird,
b) durch weitere Bewegung des Kerns die Komponente B in die pastenförmige Komponente A gepresst wird, die sich im ersten Behälter befindet,
c) danach durch axiale und/oder tangentiale Bewegung des Austragsrohrs mit der Mischeinrichtung die zweite Komponente B mit der pastenförmigen oder flüssigen ersten Komponente A zum Zementteig C vermischt wird,
d) dass während des Mischvorgangs beim Aufsetzen des Austragsrohrs mit der Mischeinrichtung auf den Austragskolben ein Sicherungsring axial auf das Austragsrohr geschoben wird bis die untere Kante des Sicherungsrings mit der unteren Kante des Austragsrohrs abschließt,
e) nach erfolgter Vermischung das Austragsrohr in Richtung des Verschlusses bewegt wird, so dass die Mischeinrichtung an der Innenseite des Volumenausgleichselements anliegt, und
f) danach der Kern aus dem Austragsrohr herausgezogen wird und dann der Austragskolben in Richtung des ersten Verschlusses bewegt wird, wobei der Zementteig C aus dem ersten Behälter durch das Austragsrohr in die Umgebung ausgepresst wird,
wobei bei den Schritten a) bis e), die Volumenänderungen des ersten Behälters durch axiale Bewegungen des Volumenausgleichselements ausgeglichen werden.

Möglich ist weiterhin, dass vor dem Herausstoßen des Stopfens die Arretierung des Volumenausgleichelements entfernt wird.

Im Folgenden werden weitere Ausführungsbeispiele der Erfindung anhand von fünf schematisch dargestellten Figuren erläutert, ohne jedoch dabei die Erfindung zu beschränken. Dabei zeigt:
- Figur 1:: eine schematische Querschnittansicht einer erfindungsgemäßen Vorrichtung mit einem Volumenausgleichselement an der Vorderseite (oben);
- Figur 2:: eine schematische Querschnittansicht einer weiteren erfindungsgemäßen Vorrichtung bei der ein Austragskolben als Volumenausgleichselement fungiert;
- Figur 3:: eine perspektivische Ansicht einer erfindungsgemäßen Vorrichtung mit einem Volumenausgleichselement an der Vorderseite (oben);
- Figur 4:: eine vergrößerte Darstellung einer Teilansicht der perspektivischen Ansicht nach Figur 3; und
- Figur 5:: eine perspektivische Ansicht des ins Innere des ersten Behälters weisenden Endes des Austragrohrs mit separatem Sicherungsring.

In den Figuren werden teilweise für gleiche oder ähnliche Bauteile der Einfachheit halber die gleichen Bezugszeichen verwendet. Geschnittene Flächen sind schraffiert dargestellt.

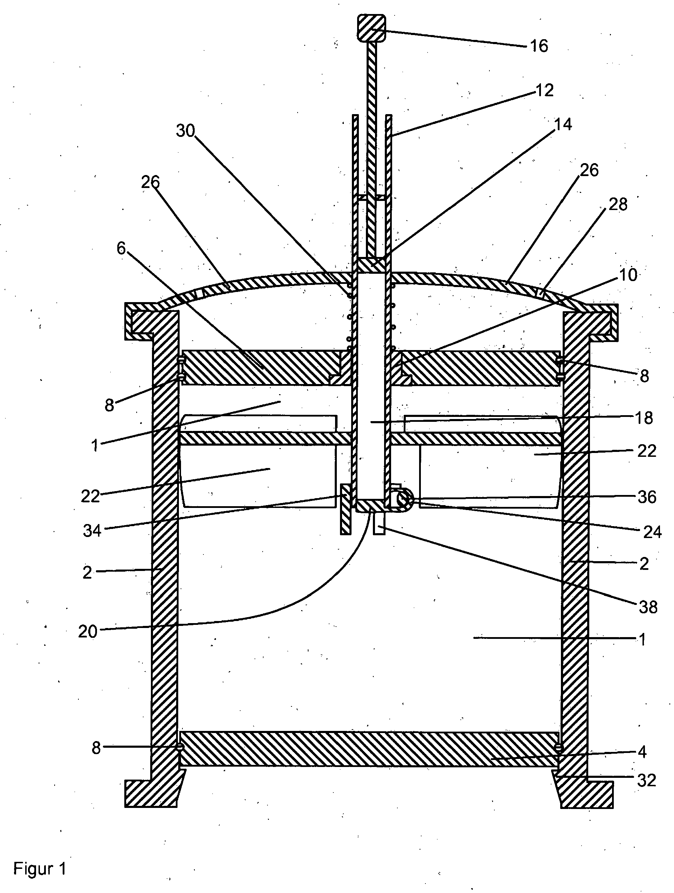

Figur 1 zeigt eine schematische Querschnittansicht einer erfindungsgemäßen Vorrichtung. Die Vorrichtung hat einen ersten Behälter 1 mit zylindrischem Innenraum, der mit einer ersten Komponente eines PMMA-Knochenzements gefüllt ist oder befüllbar ist. Als erste Komponente enthält der erste Behälter 1 eine pastöse Masse, in der das Monomer Methylmethacrylat enthalten ist. Der erste Behälter 1 ist seitlich durch die Wandungen 2 eines zylindrischen Kunststoffkörpers begrenzt. Auf der Rückseite (in Figur 1 unten) ist der erste Behälter 1 durch einen zylindrischen Austragskolben 4 begrenzt. Der erste Behälter 1 ist auf der Vorderseite (in Figur 1 oben) von einem Volumenausgleichselement 6 in Form eines zylindrischen Kolbens begrenzt. Der Austragskolben 4 und der Volumenausgleichskolben 6 sind in Richtung der Zylinderachse (in Figur 1 von oben nach unten) beweglich innerhalb der Wandungen 2 angeordnet und gegen die Wandungen 2 mit Hilfe von O-Ringen als Dichtungen 8 gasdicht abgedichtet.

In dem Volumenausgleichskolben 6 ist eine Durchführung mit einer Führungshülse 10 vorgesehen, durch die sich ein zylindrisches Austragsrohr 12 erstreckt. Das Austragsrohr 12 hat ein ins Innere des ersten Behälters 1 reichendes erstes Ende und ein gegenüberliegendes zweites Ende mit einer Austragsöffnung, über die ein fertig gemischter Zementteig appliziert werden kann. Das Austragsrohr 12 ist entlang der Zylinderachse und drehbar um die Zylinderachse beweglich in dem Volumenausgleichskolben 6 beziehungsweise der Führungshülse 10 angeordnet. Das Austragsrohr 12 ist in seinem Inneren durch einen herauslösbaren Kern 14 verschlossen.

Der Kern 14 ist über eine Stange, die sich durch das Austragsrohr 12 und die Austragsöffnung erstreckt, mit einem Griffstück 16 verbunden. Die Stange ist in der schematischen Figur 1 verkürzt dargestellt und ist tatsächlich zumindest so lange, dass der Kern 14 bis zum ersten Ende des Austragsrohrs 12 durchgeschoben werden kann, um den Inhalt des Austragsrohrs 12 darunter in den ersten Behälter 1 einschieben zu können. Zudem ist eine weitere Zylinderscheibe an der Stange zwischen dem Kern und dem Griffstück 16 vorgesehen, die der Lagerung und Stabilisierung der Bewegung der Stange im Austragsrohr 12 dient. Mit dem Griffstück 16 lässt sich das Austragsrohr 12 durch die Führungshülse 10 und damit das Austragsrohr 12 innerhalb des ersten Behälters 1 bewegen.

Unterhalb des Kerns 14 ist ein zweiter Behälter 18 in dem Austragsrohr 12 angeordnet, der auf der Rückseite (in Figur 1 unten) durch einen Verschluss 20 in Form eines Pfropfens 20 verschlossen ist. Innerhalb des zweiten Behälters 18 ist eine zweite Komponente des Knochenzements enthalten oder dort einfüllbar. Die zweite Komponente ist eine pastöse Masse oder ein Pulver. Die zweite Komponente führt zu einem radikalischen Aushärten des Knochenzements, wenn sie mit der ersten Komponente (Methylmethacrylat-Teig) gemischt wird. Durch den Pfropfen 20 beziehungsweise den Verschluss 20 und das Austragsrohr 12 sind die beiden Komponenten zunächst voneinander getrennt.

Außen an dem Austragsrohr 12 sind Mischflügel 22 angeordnet, mit denen der Inhalt des ersten Behälters 1 durch Bewegen des Austragsrohrs 12 in der Führungshülse 10 durchmischt werden kann. Die Mischflügel 22 bilden damit eine Mischeinrichtung 22 zum manuellen Durchmischen des Inhalts des ersten Behälters 1. Die Mischflügel 22 sind vorliegend nach Art eines Propellers gegenläufig geneigt.

Der Pfropfen 20 ist über einen gebogenen und gespannten Steg 24 mit dem ersten Ende des Austragsrohrs 12 verbunden. Der zweite Behälter 18 kann geöffnet werden, indem der Kern 14 in Richtung des ersten Behälters 1 in das Austragsrohr 12 hinein geschoben wird. Der Inhalt des zweiten Behälters 18 (die zweite Komponente) vermittelt den Druck auf den Pfropfen 22, bis der Pfropfen 22 sich löst und durch den gespannten Steg 24 von der Öffnung am ersten Ende des Austragsrohrs 12 weg gekippt beziehungsweise bewegt wird.

Die beiden fluiden Komponenten sind in den beiden Behältern 1, 18 ohne Lufteinschlüsse enthalten beziehungsweise werden ohne Gas- oder Lufteinschlüsse dort eingebracht. Wenn die zweite Komponente ein Pulver ist, ist in den Zwischenräumen Gas, vorzugsweise ein steriles oder sterilisierendes Gas enthalten. Zudem wird das Innere der Behälter 1, 18 vor dem Befüllen entgast und sterilisiert. Da sich kein Gas im Inneren des ersten Behälters 1 befindet, ist der Inhalt des ersten Behälters 1 inkompressibel. Durch das Einschieben oder Herausziehen des Austragsrohrs 12 verändert sich dann das Volumen des Inhalts des ersten Behälters 1. Der Ausgleich des Volumens kann durch eine axiale Bewegung des Volumenausgleichskolbens 6 und, wenn gewünscht und entsprechend konstruiert, auch durch eine axiale Bewegung des Austragskolbens 4 erfolgen. Dadurch ist eine Durchmischung des Inhalts des ersten Behälters 1 möglich, ohne dass der Inhalt des ersten Behälters 1 austritt oder Luft in den ersten Behälter 1 eingezogen wird.

Auf der Vorderseite (in Figur 1 oben) ist der äußere zylindrische Kunststoffkörper 2 mit einem Deckel 26 verschlossen. In dem Deckel 26 sind Öffnungen 28 vorgesehen, damit Luft aus dem Zwischenraum zwischen dem Deckel 26 und dem Volumenausgleichskolben 6 entweichen kann. Die Öffnungen 28 können auch weggelassen werden, damit besagter Zwischenraum als Gasfeder fungieren kann. Bevorzugt ist jedoch zwischen dem Deckel 26 und dem Volumenausgleichskolben 6 eine Stahlfeder 30 als Rückstellelement angeordnet. Wenn der Volumenausgleichskolben 6 in Folge eines Einschiebens des Austragsrohrs 12 in den ersten Behälter 1 in Richtung des Deckels 26 gedrückt wird, hilft die Stahlfeder 30 dabei, den Volumenausgleichskolben 6 wieder in Richtung des Austragskolbens 4 zu drücken, wenn das Austragsrohr 12 wieder aus dem ersten Behälter 1 herausgezogen wird. Ein Arretierungsmittel (nicht gezeigt) kann vorgesehen sein, um den Volumenausgleichskolben 6 zu sperren, so dass dieser sich nicht beim Befüllen des ersten Behälters 1 mit der ersten Komponente bewegen kann. Bevor die zweite Komponente in den ersten Behälter 1 gefüllt wird oder bevor die beiden Komponenten im ersten Behälter 1 gemischt werden, kann das Arretierungsmittel manuell gelöst werden.

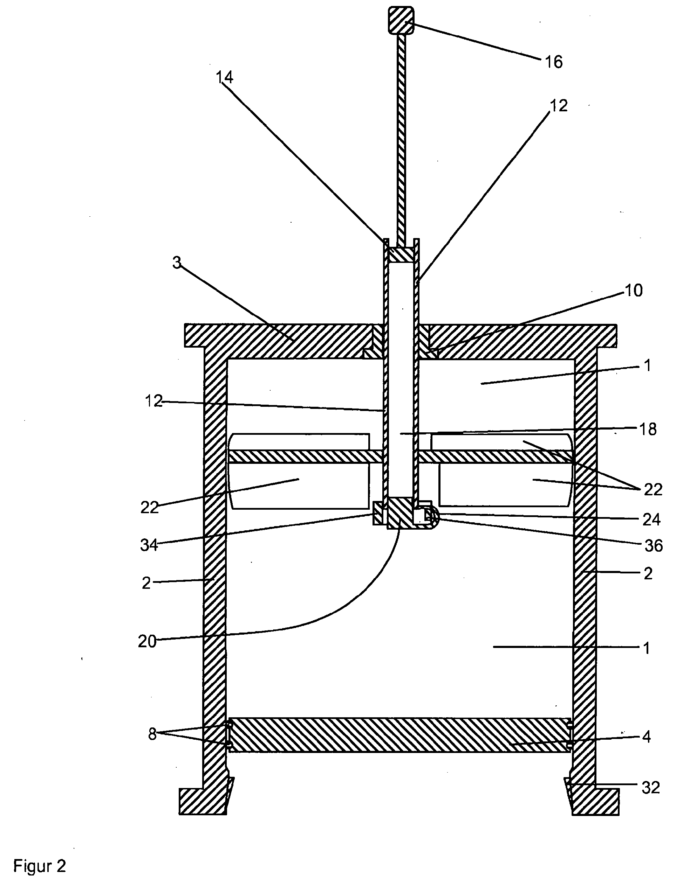

Zum Einschieben der zweiten Komponente in den ersten Behälter 1 sollte die Stange, über die der Kern 14 mit dem Griffstück 16 verbunden ist, lange genug sein (also länger als in der schematischen Figur 1 und auch in den Figuren 2, 3 und 4 dargestellt). Dadurch kann sichergestellt werden, dass der gesamte Inhalt des zweiten Behälters 18 in den ersten Behälter 1 überführt werden kann. Mit einer Abstreiflippe (nicht gezeigt), die an dem unteren Rand des Kerns 14 vorgesehen ist, kann sichergestellt werden, dass der gesamte Inhalt des zweiten Behälters 18 ohne Rückstände aus dem Austragsrohr 12 entfernt werden kann.

An der Unterseite des Kunststoffkörpers 2 sind Rastelemente 32 angeordnet, mit denen sichergestellt werden kann, dass der Austragskolben 4 nicht nach unten aus dem Kunststoffkörper 2 herausgedrückt werden kann. Beim Einschieben des Austragskolbens 4 werden diese kurzzeitig in die Wandung versenkt und/oder die Rastelemente 32 verformen kurzzeitig die Dichtung 8 und gegebenenfalls sogar den Austragskolben 4. Ein kleiner Vorsprung, der zu einer stärkeren Stauchung der Dichtungen 8 des Austragskolbens 4 führt, kann ebenfalls an der Unterseite der Wandungen 2 vorgesehen sein.

Ein Sicherungsring 34 ist auf das erste Ende des Austragsrohrs 12 geschoben. Eine Rastung (nicht gezeigt) am Sicherungsring 34 und eine Gegenrastung (nicht gezeigt) am Austragsrohr 12 sorgen dafür, dass sich der Sicherungsring 34 nur noch weiter auf das erste Ende des Austragsrohrs 12 schieben lässt und sich nicht ungewollt von dem ersten Ende des Austragsrohrs 12 lösen kann.

Auf einer Seite (in Figur 1 rechts) weist der Sicherungsring 34 eine Strebe 36 auf, die eine radiale Ausnehmung auf der Innenseite des Sicherungsrings 34 überbrückt. Der Steg 24, mit dem der Pfropfen 20 an dem Austragsrohr 12 befestigt ist, ist außen um diese Strebe 36 herum gebogen. Die radiale Ausnehmung auf der Innenseite des Sicherungsrings 34, die die Strebe 36 überbrückt, ist so breit und so tief, dass sie den Steg 24 aufnehmen und umbiegen kann. An der dem Austragskolben 4 zugewandten Seite des Sicherungsrings 34 sind mehrere Verlängerungsstifte 38 vorgesehen, die in Richtung des Austragskolbens 4 über das erste Ende des Austragsrohrs 12 hinausragen. Der Sicherungsring 34 hat damit zwischen zwei Verlängerungsstiften 38 eine axiale Ausnehmung in Richtung des Austragskolbens 4 (in Figur 1 rechts), durch die sich der Steg 24 erstreckt.

Wenn der Inhalt des zweiten Behälters 18 mit dem Kern 14 in den ersten Behälter 1 gedrückt ist, wird das Austragsrohr 12 zum Durchmischen der beiden Komponenten auch axial bewegt. Wenn das Austragsrohr 12 bis zum Austragskolben 4 geschoben wird, wird der Sicherungsring 34 auf das Austragsrohr 12 aufgeschoben, bis die Enden der Verlängerungsstifte 38 bündig mit dem ersten Ende des Austragsrohrs 12 sind. Dabei schiebt sich die Strebe 36 über den Steg 24 und biegt diesen um, so dass er zumindest bereichsweise an der Außenwand des Austragsrohrs 12 anliegt und sich von seiner Verbindung zum Austragsrohr 12 in Richtung der Vorderseite der Vorrichtung (in Figur 1 oben) erstreckt.

Bevorzugt ist eine zweite Gegenrastung (nicht gezeigt) vorgesehen, die in die Rastung des Sicherungsrings 34 greift, so dass der Sicherungsring 34 in dieser eingeschobenen Position verbleibt. Hierdurch wird erreicht, dass der geöffnete Pfropfen 20 sich nicht mehr bis zur Öffnung des Austragsrohr 12 an dessen erstem Ende bewegen kann und dadurch die Öffnung verschließt oder deren freien Querschnitt und damit eine mögliche Strömung in das Austragsrohr 12 hinein erschwert.

Figur 2 zeigt eine schematische Querschnittansicht einer weiteren erfindungsgemäßen Vorrichtung bei der der Austragskolben 4 alleine als Volumenausgleichselement 4 fungiert. Der Aufbau der Vorrichtung gleicht der nach Figur 2 bis auf einige Details.

Die Vorrichtung nach Figur 2 hat keinen zusätzlichen Volumenausgleichskolben und auch keinen Deckel. Stattdessen ist der Kunststoffkörper 2 einfach auf der Vorderseite (in Figur 2 oben) geschlossen und bildet dort eine Deckwand 3. Der Volumenausgleich durch das Einschieben und Herausziehen des Austragsrohrs 12 erfolgt hier ausschließlich durch eine axiale Bewegung des Austragskolbens 4. Auf ein Federelement als Rückstellelement wurde bei dieser Ausführungsform verzichtet.

Der Sicherungsring 34 weist bei dieser Ausführung keine Verlängerungsstifte auf sondern ist über seine Wandungen vorliegend derart tief ausgestaltet, dass er selbst weit genug über das erste Ende des Austragsrohrs 12 hinausreicht, so dass er durch Aufdrücken des Austragsrohrs 12 auf den Austragskolben 4 aufgeschoben werden kann und dadurch der Steg 24 an das Austragsrohr 12 angepresst und der Pfropfen 20 gesichert wird. Die axiale Ausnehmung im Sicherungsring 34 erstreckt sich in diesem Fall in die Wandungen der Sicherungsrings 34 hinein. Zudem ist an dieser Stelle auch wieder eine radiale Ausnehmung am inneren Umfang des Sicherungsrings 34 vorhanden. Die verbleibende Strebe 36, die den Sicherungsring 34 an dieser Stelle schließt, ist über den Außenumfang des restlichen Sicherungsrings 34 erhaben und wie zu dem Ausführungsbeispiel nach Figur 1 dazu geeignet, den Steg 24 umzubiegen und dadurch den Pfropfen 20 von der Öffnung des Austragsrohrs 12 am ersten Ende fernzuhalten.

Der Austragskolben 4 ist vorliegend mit zwei Dichtungen 8 gegen die Wandungen 2 gasdicht abgedichtet. Zu allen anderen Bezugszeichen wird auf die Beschreibung zu Figur 1 verwiesen.

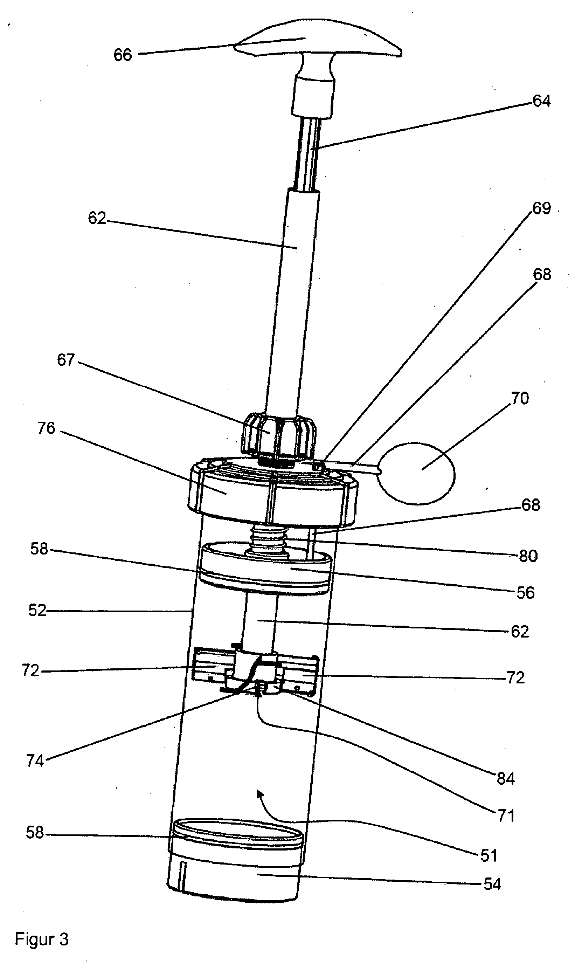

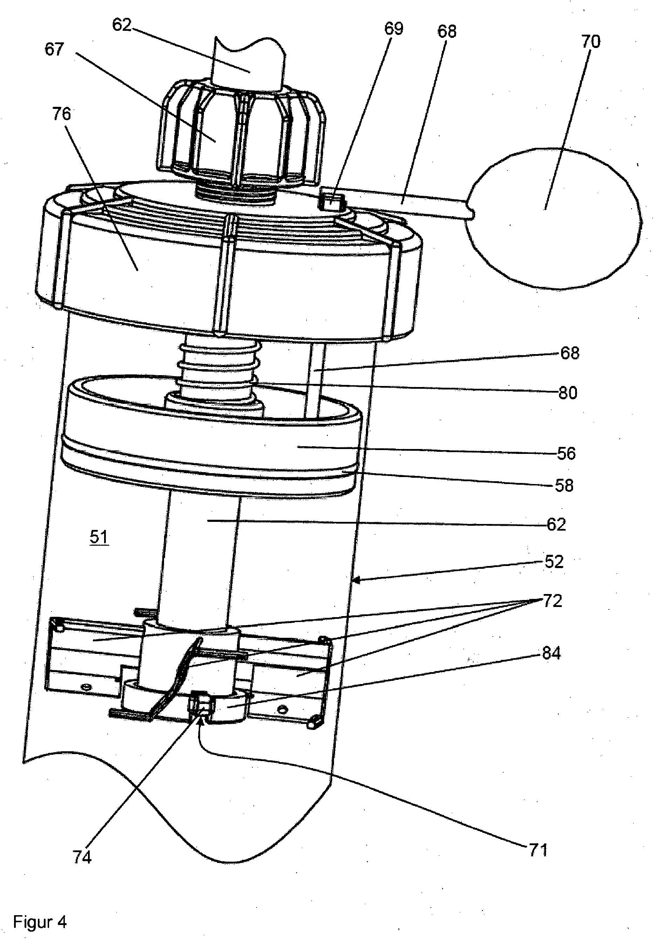

Figur 3 zeigt eine perspektivische Ansicht einer erfindungsgemäßen Vorrichtung mit einem Volumenausgleichselement 56 an der Vorderseite (oben) und Figur 4 zeigt eine vergrößerte Darstellung einer Teilansicht der perspektivischen Ansicht nach Figur 3. Die in den Figuren 3 und 4 gezeigte Vorrichtung zur Lagerung, Vermischung und Applikation von Polymethylmethacrylat-Knochenzement hat einen ersten Behälter 51 mit zylindrischem Innenraum, der durch transparente Wandungen 52 begrenzt ist. Auf der Rückseite (in den Figuren 3 und 4 unten) ist der Innenraum des ersten Behälters 51 mit einem zylindrischen Austragskolben 54 (in Figur 4 nicht zu sehen) abgeschlossen und auf der Vorderseite (in Figur 3 und 4 oben) ist der Innenraum des ersten Behälters 51 mit einem zylindrischen Volumenausgleichskolben 56 begrenzt. Der Austragskolben 54 und der Volumenausgleichskolben 56 sind entlang der Zylinderachse (in den Figuren 3 und 4 von oben nach unten) beweglich im Innenraum des ersten Behälters 51 angeordnet und mit je einer umlaufenden Dichtung 58 gegen die Innenwand des ersten Behälters 51 abgedichtet.

Die Vorderseite des ersten Behälters 51 ist mit einem Deckel 76 geschlossen. Durch eine Durchführung auf der Zylinderachse des Volumenausgleichskolbens 56 und durch eine Durchführung durch den Deckel 76 ragt ein Austragsrohr 62, das sich aus dem Inneren des ersten Behälters 51 zur Vorderseite hinaus erstreckt und dort in eine Austragsöffnung mündet. In dem Austragsrohr 62 ist ein Kern (in den Figuren 3 und 4 nicht zu sehen) angeordnet, der das Austragsrohr 62 in Richtung der Vorderseite verschließt. Der Kern ist an der Spitze einer Stange 64 befestigt, an deren anderem Ende ein Griffstück 66 befestigt ist. Mit Hilfe des Griffstücks 66 ist der Kern im Inneren des Austragsrohrs 62 beweglich. Mit Hilfe einer Überwurfmutter 67 kann das Austragsrohr 62 gegen den Deckel 76 fixiert und gelöst werden. Ansonsten ist das Austragsrohr 62 in Längsrichtung beweglich und drehbar gegenüber dem ersten Behälter 51 gelagert.

Eine Arretierung 68 reicht durch eine Öffnung im Deckel 76 bis zum Volumenausgleichskolben 56. Über einen Haken 69 und eine entsprechend passende Gegenrastung an der Arretierung 68 ist die Arretierung 68 gegen den Deckel 76 fixiert, so dass der Volumenausgleichskolben 56 nicht in Richtung des Deckels 76 oder sogar gar nicht relativ zum Deckel 76 und damit zu den Wandungen 52 bewegt werden kann. Über einen Griff 70 der Arretierung 68 kann die Gegenrastung der Arretierung 68 von dem Haken 69 des Deckels 76 gelöst werden und anschließend entfernt werden. Der Volumenausgleichskolben 56 ist dann wieder gegen den Deckel 76 voll beweglich. Das Arretieren des Volumenausgleichskolbens 56 dient dazu, eine Bewegung beim Befüllen des ersten Behälters 51 mit der ersten Komponente und beim Evakuieren und Sterilisieren des ersten Behälters 51 zu verhindern und diese Vorgänge damit zu erleichtern.

Die ins Innere des ersten Behälters 51 weisende Seite des Austragsrohrs 62 ist mit einem Verschluss 71 verschlossen. Die Innenwände des Austragsrohrs 62 bilden zusammen mit dem Kern und dem Verschluss 71 einen zweiten Behälter, der für die zweite Komponente eines Zements vorgesehen ist oder der mit der zweiten Komponente gefüllt ist. Dieses Ende (auch als erstes Ende bezeichnet) des Austragsrohrs 62 wird durch eine kurze Hülse gebildet, die fest mit dem restlichen Austragsrohr 62 verbunden ist. An dieser Hülse sind vier Mischflügel 72 befestigt, die sich durch eine Bewegung des Austragsrohrs 62 im Inneren der ersten Behälters 51 bewegen lassen und die als Mischeinrichtung 72 für den Inhalt des ersten Behälters 51 dienen.

Der Verschluss 71 ist über einen flexiblen, elastischen, vorgespannten Steg 74 mit der Hülse und damit mit dem Austragsrohr 62 verbunden. Wenn also mit dem Kern ein Druck auf den Verschluss 71 ausgeübt wird, der über die zweite Komponente vermittelt wird, schiebt sich der Verschluss 71 aus dem Ende des Austragsrohrs 62 heraus und wird von dem Steg 74, der eine gerade Form (im entspannten Zustand) anstrebt, von der Öffnung weg bewegt. Anschließend kann der Inhalt des zweiten Behälters durch Vortreiben des Kerns in den ersten Behälter 51 überführt werden.

Zwischen dem Deckel 76 und dem Volumenausgleichskolben 56 ist eine elastische Feder 80 um das Austragsrohr 62 herum angeordnet, die im kräftefreien Zustand einen bestimmten Abstand zwischen dem Volumenausgleichskolben 56 und dem Deckel 76 herstellt.

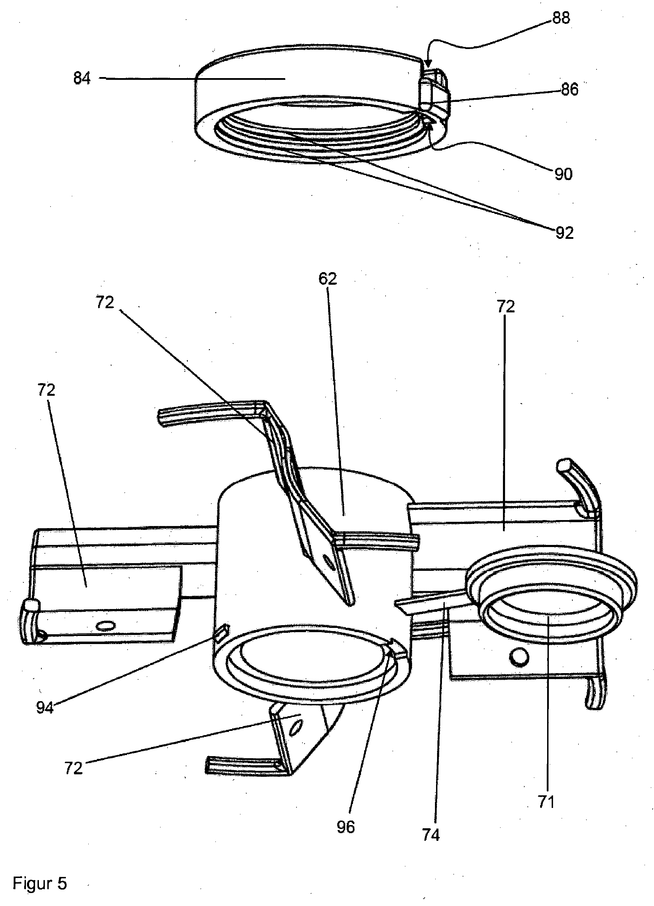

Ein Sicherungsring 84 ist als Sicherungselement auf das nach Innen weisende erste Ende des Austragsrohrs 62 beziehungsweise die Hülse aufgesteckt und dort eingerastet. Der Steg 74 ist um eine Strebe 86 des Sicherungsrings 84 herum gebogen, die in der Einzelansicht nach Figur 5 zu erkennen ist.

Mit Hilfe der Mischeinrichtung 72 können die beiden Komponenten im Inneren des ersten Behälters 51 gemischt werden. Um eine ausreichende Mischung zu erreichen, muss die Mischeinrichtung 72 im gesamten Volumen des ersten Behälters 51 betätigt werden und also durch Herausziehen und Hineinschieben des Austragsrohrs 62 bewegt werden. Dabei wird sich das Volumen des Inhalts des ersten Behälters 51 ändern. Diese Volumenänderung wird durch die Bewegung des Volumenausgleichskolbens 56 aufgenommen, da die Komponenten in der Zement-Mischung inkompressibel sind und kein Gas oder nur sehr wenig Gas in der Zement-Mischung enthalten ist.

Gleichzeitig wird dabei das erste Ende des Austragsrohrs 62 auf den Austragskolben 58 gedrückt und so der Sicherungsring 84 weiter aus das Austragsrohr 62 aufgeschoben. Dadurch wird der Steg 74 mit Hilfe der Strebe 86 von der Öffnung des Austragsrohrs 62 weg gedrückt und damit auch der Verschluss 71. Dadurch wird sichergestellt, dass der Verschluss 71 sich nicht später störend auf das Austragen des Zementgemischs auswirkt.

Das fertig gemischte Zementgemisch wird aus dem ersten Behälter 51 ausgetrieben, indem der Austragskolben 54 in Richtung des Deckels 76 gedrückt wird. Dies kann mit einer geeigneten Auspresspistole und/oder durch Druckluft geschehen. Zuvor wird das Austragsrohr 62 nach vorne, so weit es geht aus dem ersten Behälter 51 herausgezogen, so dass die Mischflügel 72 innen an dem Volumenausgleichskolben 56 anliegen, und der Kern mit Hilfe des Griffstücks 66 aus dem Austragsrohr heraus gezogen und damit die Vorrichtung geöffnet.

Eine erfindungsgemäße Vorrichtung ist also eine Zementkartusche zum Lagern, Mischen und Applizieren eines Zweikomponenten-Zements.

Figur 5 zeigt eine perspektivische Ansicht des ins Innere des ersten Behälters 51 weisenden Endes des Austragrohrs 62 mit separat angeordnetem Sicherungsring 84. Der Sicherungsring 84 (in Figur 5 oben) hat eine axiale Ausnehmung 88 und eine radiale Ausnehmung 90, die von der Strebe 86 überbrückt werden. In der Innenwand des Sicherungsrings 84 sind zwei Rinnen 92 oder Rillen 92 angeordnet, die als Gegenrastung für eine Rastmittel 94 an der Außenwand des Austragsrohrs 62 dienen.

Das Ende des Austragsrohrs 62 (beziehungsweise die Hülse) weist an der Unterseite eine axiale Ausnehmung 96 auf, in die sich der Steg 74 legen kann, wenn der Verschluss 71 die Öffnung des Austragsrohrs 62 verschließt. In dem in Figur 5 gezeigten Zustand ist der Steg 74 entspannt. Bei Zusammenbau der Vorrichtung wird der Sicherungsring 84 bezüglich der in Figur 5 gezeigten Orientierung senkrecht zur Symmetrieachse um 180° gedreht und auf das gezeigte erste Ende des Austragrohrs 62 von unten aufgesteckt, bis das Rastmittel 94 in die erste zugänglichen Rinne 92 greift. Die Strebe 86 liegt dann auf dem Steg 74 auf. Um eine fehlerhafte Orientierung des Sicherungsrings zu vermeiden, kann eine geeignete Indexierung des ersten Endes des Austragsrohrs 62 und des Sicherungsrings 84 vorgesehen sein. Anschließend wird der Verschluss 71 um 180° umgeklappt und in die Öffnung des Austragsrohrs 62 gesteckt. Dabei wird der flexible, elastische Steg 74 um die Strebe 86 herum gebogen. Dann ergibt sich der in den Figuren 3 und 4 gezeigte Zustand der Vorrichtung.

Die wesentlichen Aufbauten nach den Figuren 1 bis 5 können einfach und kostengünstig mit Spritzgusstechnik aus Kunststoff gefertigt werden. Als Federelemente beziehungsweise Federn werden bevorzugt Stahlfedern verwendet. Die Füllungen des ersten Behälters 1 und des zweiten Behälters 18 bestehen selbstverständlich nicht aus Kunststoff sondern aus den Ausgangskomponenten des medizinischen Zements.

### Bezugszeichenliste

- 1, 51: Erster Behälter
- 2, 52: Wandung
- 3: Deckwand
- 4, 54: Austragskolben
- 6, 56: Volumenausgleichskolben / Volumenausgleichselement
- 8,58: Dichtung
- 10: Führungshülse
- 12, 62: Austragsrohr
- 14: Kern
- 16, 66: Griffstück
- 18: Zweiter Behälter
- 20, 71: Verschluss / Pfropfen
- 22, 72: Mischflügel / Mischeinrichtung
- 24, 74: Steg
- 26, 76: Deckel
- 28: Öffnung
- 30, 80: Feder
- 32: Rastung
- 34, 84: Sicherungsring
- 36, 86: Strebe
- 38: Verlängerungsstift
- 64: Stange
- 67: Überwurfmutter
- 68: Arretierung
- 69: Haken / Rastmittel
- 70: Griff der Arretierung
- 88: Axiale Ausnehmung
- 90: Radiale Ausnehmung
- 92: Rinne / Gegenrastung
- 94: Rastmittel
- 96: Axiale Ausnehmung

## Patentansprüche

1. Vorrichtung zur Lagerung, Vermischung und Applikation von Polymethylmethacrylat-Knochenzement aufweisend einen ersten Behälter (1, 51) für eine erste pastenförmige Komponente des Knochenzements, einen im ersten Behälter (1, 51) verschiebbar angeordneten Austragskolben (4, 54) zum Auspressen des Inhalts des ersten Behälters (1, 51) durch ein dem Austragskolben (4, 54) gegenüberliegendes Austragsrohr (12, 62), wobei das Austragsrohr (12, 62) drehbar und in Längsrichtung verschiebbar durch eine Durchführung in einer dem Austragskolben (4, 54) gegenüberliegenden Seite des Behälters (1, 51) angeordnet ist, und eine Mischeinrichtung (22, 72) zum Durchmischen des Inhalts des ersten Behälters (1, 51), wobei die Mischeinrichtung (22, 72) im ersten Behälter (1, 51) angeordnet ist und an dem Austragsrohr (12, 62) befestigt ist, so dass die Mischeinrichtung (22, 72) im ersten Behälter (1, 51) durch Bewegen des Austragsrohrs (12, 62) zum Durchmischen des Inhalts des ersten Behälters (1, 51) bewegbar ist, wobei an dem ins Innere des ersten Behälters (1, 51) weisende ersten Ende des Austragsrohrs (12, 62) ein zu öffnender Verschluss (20, 71) angeordnet ist, der das Austragsrohr (12, 62) verschließt, und in dem Austragsrohr (12, 62) ein axial beweglicher Kern (14) angeordnet ist, und ein zweiter Behälter (18) für zumindest eine zweite Komponente des Knochenzements durch den Raum zwischen dem Verschluss (20, 71) und dem Kern (14) im Austragsrohr (12, 62) gebildet ist, wobei der Verschluss (20, 71) des zweiten Behälters (18) durch eine axiale Bewegung des Kerns (14) von dem ersten Ende des Austragsrohrs (12, 62) entfernbar ist und dadurch der zweite Behälter (18) zum ersten Behälter (1, 51) zu öffnen ist, so dass die Inhalte des ersten Behälters (1, 51) und des zweiten Behälters (18) im ersten Behälter (1, 51) miteinander mischbar sind, wobei zumindest eine Begrenzungsfläche des ersten Behälters (1, 51) durch ein bewegliches Volumenausgleichselement (4, 6, 56) gebildet ist und der Verschluss (20, 71) über eine verformbare Verbindung (24, 74) mit dem Austragsrohr (12, 62) verbunden ist, so dass auch der geöffnete Verschluss (20, 71) über die verformbare Verbindung (24, 74) mit dem Austragsrohr (12, 62) verbunden ist, **dadurch gekennzeichnet, dass** an dem ins Innere weisenden ersten Ende des Austragsrohrs (12, 62) ein Sicherungselement (34, 84) angeordnet ist und mit dem Sicherungselement (34, 84) der geöffnete Verschluss (20, 71) arretierbar oder derart in seiner Beweglichkeit gegenüber der Öffnung des ersten Endes des Austragsrohrs (12, 62) einzuschränken ist, dass die Öffnung des ersten Endes des Austragsrohrs (12, 62) durch den Verschluss (20, 71) nicht mehr verschließbar ist oder zu verkleinern ist.

2. Vorrichtung nach Anspruch 1, **dadurch gekennzeichnet, dass** der Verschluss (20, 71) eine Kappe ist, die auf dem ins Innere weisende ersten Ende des Austragsrohrs (12, 62) steckt oder angeordnet ist, oder der Verschluss (20, 71) ein Stopfen (20, 71) ist, der in dem ins Innere weisende ersten Ende des Austragsrohrs (12, 62) steckt oder angeordnet ist.

3. Vorrichtung nach Anspruch 1 oder 2, **dadurch gekennzeichnet, dass** die verformbare Verbindung (24, 74) ein Steg (24, 74) ist, der gebogen ist, wenn der Verschluss (20, 71) das Austragsrohr (12, 62) verschließt, wobei vorzugsweise der Steg (24, 74) und der Verschluss (20, 71) einteilig ausgeführt sind.

4. Vorrichtung nach einem der vorangehenden Ansprüche, **dadurch gekennzeichnet, dass**
die verformbare Verbindung (24, 74) gespannt ist und eine Federkraft auf den Verschluss (20, 71) bewirkt, die den Verschluss (20, 71) von der Öffnung des ersten Endes des Austragsrohrs (12, 62) weg bewegt, wenn der Verschluss (20, 71) durch die axiale Bewegung des Kerns (14) von dem ersten Ende des Austragsrohrs (12, 62) gelöst ist.

5. Vorrichtung nach einem der vorangehenden Ansprüche, **dadurch gekennzeichnet, dass**
das Sicherungselement (34, 84) ein Sicherungsring (34, 84) ist, der auf das erste Ende des Austragsrohrs (12, 62) aufgesteckt ist, der in seiner Ausgangslage über das erste Ende hinaus ragt und der weiter auf das erste Ende des Austragsrohrs (12, 62) aufschiebbar ist, wobei das Aufschieben des Sicherungsrings (34, 84) den Verschluss (20, 71) sichert, in dem durch die neue Lage des Sicherungsrings (34, 84) die Deformierbarkeit der verformbaren Verbindung (24, 74) eingeschränkt ist.

6. Vorrichtung nach einem der vorangehenden Ansprüche, **dadurch gekennzeichnet, dass**
der Austragskolben (4, 54) gegen den ersten Behälter (1, 51) arretierbar ist oder arretiert ist, bevorzugt an dem dem Austragsrohr (12, 62) gegenüberliegenden Ende des ersten Behälters (1, 51).

7. Vorrichtung nach einem der vorangehenden Ansprüche, **dadurch gekennzeichnet, dass**
der erste Behälter (1, 51) einen zylindrischen Innenraum aufweist und der Austragskolben (4, 54) in dem Innenraum des ersten Behälters (1, 51) eine Passform entsprechend der Grundfläche des zylindrischen Innenraums aufweist.

8. Vorrichtung nach einem der vorangehenden Ansprüche, **dadurch gekennzeichnet, dass**
zumindest ein Volumenausgleichselement (4, 6, 56) axial beweglich in oder an dem ersten Behälter (1, 51) angeordnet ist.

9. Vorrichtung nach Anspruch 8, **dadurch gekennzeichnet, dass**
das zumindest eine Volumenausgleichselement (6, 56) die gasdichte Durchführung umfasst, durch die das Austragsrohr (12, 62) zum Bedienen der Mischeinrichtung (22, 72) hindurchgeführt ist.

10. Vorrichtung nach einem der vorangehenden Ansprüche, **dadurch gekennzeichnet, dass**
ein Volumenausgleichselement (4) durch den Austragskolben (4) realisiert ist und die Bewegung des Austragskolbens (4) aus dem ersten Behälter (1) heraus durch ein Begrenzungselement (32) begrenzt ist, wobei das Begrenzungselement (32) bevorzugt eine Rastung (32) ist, die in eine Gegenrastung am Austragskolben (4) greift.

11. Vorrichtung nach einem der vorangehenden Ansprüche, **dadurch gekennzeichnet, dass**
ein Volumenausgleichselement (6, 56) gegenüber dem Austragskolben (4, 54) in dem ersten Behälter (1, 51) als beweglich gelagertes Volumenausgleichselement (6, 56) angeordnet ist, wobei bevorzugt das Volumenausgleichselement (6, 56) die gasdichte Durchführung für das Austragsrohr (12, 62) umfasst.

12. Vorrichtung nach einem der vorangehenden Ansprüche, **dadurch gekennzeichnet, dass**
der Kern (14) als Verschlusselement der Austragöffnung des Austragsrohrs (12, 62) vorgesehen ist, wobei die Austragöffnung der ersten Öffnung gegenüberliegend angeordnet ist, wobei der Kern (14) aus dem Austragsrohr (12, 62) herausnehmbar ist.

13. Vorrichtung nach einem der vorangehenden Ansprüche, **dadurch gekennzeichnet, dass**
der Kern (14) an der dem ersten Behälter (1, 51) zugewandten Seite einen Abstreifer aufweist, der beim Herausziehen des Kerns (14) Pulver oder Zementteig an der Innenseite des Austragsrohrs (12, 62) abstreift.

14. Vorrichtung nach einem der vorangehenden Ansprüche, **dadurch gekennzeichnet, dass**
zumindest ein Volumenausgleichselement (6, 56) über eine elastische Feder (30, 80) gegen den ersten Behälter (1, 51) beweglich gelagert ist, wobei die Feder (30, 80) das Volumenausgleichselement (6, 56) in den Innenraum des ersten Behälters (1, 51) drückt.

15. Vorrichtung nach einem der vorangehenden Ansprüche, **dadurch gekennzeichnet, dass**
die Seite des Behälters (1, 51) mit dem Austragsrohr (12, 62) von einer Verschlusskappe (26, 76) verschlossen ist, die eine Durchführung für das Austragsrohr (12, 62) aufweist, die ein Volumenausgleichselement (6, 56) abdeckt und die Verschlusskappe (26, 76) zumindest eine Öffnung (28) zum Ermöglichen eines Druckausgleichs zwischen der Umgebung und dem Zwischenraum zwischen der Verschlusskappe (26, 76) und dem abgedeckten Volumenausgleichselement (6, 56) aufweist, wobei bevorzugt eine elastische Spiralfeder (30, 80) zum Drücken des Volumenausgleichselements (6, 56) in den Innenraum des ersten Behälters (1, 51) zwischen der Verschlusskappe (26, 76) und dem abgedeckten Volumenausgleichselement (6, 56) angeordnet ist, wobei besonders bevorzugt die Spiralfeder (30, 80) um das Austragsrohr (12, 62) herum angeordnet ist.

16. Vorrichtung nach einem der vorangehenden Ansprüche, **dadurch gekennzeichnet, dass**
das Volumenausgleichselement (4, 6, 56) mit einem lösbaren Arretierungsmittel arretiert ist.

17. Verfahren zum Herstellen eines Polymethylmethacrylat-Knochenzements mit einer Vorrichtung nach einem der vorangehenden Ansprüche, **gekennzeichnet durch** die Schritte:
A) Bereitstellen der Vorrichtung, wobei der erste Behälter (1, 51) mit einer ersten flüssigen oder pastenförmigen Komponente des PMMA-Knochenzements gefüllt ist und der zweite Behälter (18) mit einer zweiten, vorzugsweise pulverförmigen oder pastenförmigen Komponente des PMMA-Knochenzements gefüllt ist;
B) Öffnen des zweiten Behälters (18) **durch** Vorschieben des Kerns (14) in dem Austragsrohr (12, 62) und Austragen der zweiten Komponente aus dem zweiten Behälter (18) in den ersten Behälter (1, 51) **durch** weiteres Vortreiben des Kerns (14) in dem Austragsrohr (12, 62); und
C) Mischen der beiden Komponenten im ersten Behälter (1, 51) **durch** Bewegen der Mischeinrichtung (22, 72), wobei beim Bewegen der Mischeinrichtung (22, 72) das mit der Mischeinrichtung (22, 72) verbundene Austragsrohr (12, 62) in den ersten Behälter (1, 51) wiederholt hinein geschoben und heraus gezogen wird, wobei die Volumenveränderung des Inhalts des ersten Behälters (1, 51) beim Mischen **durch** eine Bewegung des zumindest einen Volumenausgleichselements (4, 6, 56) ausgeglichen wird, wobei das ins Innere des Behälters (1, 51) weisende erste Ende des Austragsrohrs (12, 62) bis auf den Austragkolben (4, 54) gedrückt wird, wobei **durch** den Druck ein Sicherungselement (34, 84), das an diesem ersten Ende angeordnet ist, bedient wird und mit der Bedienung des Sicherungselements (34, 84) der geöffnete Verschluss (20, 71) arretiert wird oder derart in seiner Beweglichkeit gegenüber der Öffnung des ersten Endes des Austragsrohrs (12, 62) eingeschränkt wird, dass die Öffnung des ersten Endes des Austragsrohrs (12, 62) **durch** den Verschluss (20, 71) nicht mehr verschlossen werden kann oder verkleinert werden kann.

18. Verfahren nach Anspruch 17, **dadurch gekennzeichnet, dass**
nach Schritt B) oder nach Schritt C) der Kern (14) aus dem Austragsrohr (12, 62) entfernt wird und danach ein Schritt D) erfolgt, bei dem der gemischte Knochenzement durch Vortreiben des Austragskolbens (4, 54) in dem ersten Behälter (1, 51) appliziert wird.

19. Verfahren nach einem der Ansprüche 17 bis 18, **dadurch gekennzeichnet, dass**
die Mischeinrichtung (22, 72) mit dem Austragsrohr (12, 62) verbunden ist und der Inhalt des ersten Behälters (1, 51) gemischt wird, indem die Mischeinrichtung (22, 72) durch hineinbewegen und herausbewegen des Austragsrohrs (12, 62) in den ersten Behälter (1, 51) in dem ersten Behälter (1, 51) bewegt wird, wobei bevorzugt zusätzlich die Mischeinrichtung (22, 72) durch Drehen des Austragsrohrs (12, 62) im ersten Behälter (1, 51) gedreht wird.

20. Verfahren nach einem der Ansprüche 17 bis 19, **dadurch gekennzeichnet, dass** vor Schritt A) der erste Behälter (1, 51) mit einer Komponente des Knochenzements gefüllt wird und das Innere des ersten Behälters (1, 51) zuvor entgast und sterilisiert wird.

21. Verfahren nach Anspruch 20, **dadurch gekennzeichnet, dass**
der Austragskolben (4, 54) und/oder das Volumenausgleichselement (4, 6, 56) arretiert wird, um das Innere des ersten Behälters (1, 51) zu entgasen und zu sterilisieren.

22. Verfahren nach einem der Ansprüche 17 bis 21, **dadurch gekennzeichnet, dass**
nach erfolgter Vermischung das Austragsrohr (12, 62) in Richtung aus dem ersten Behälter (1, 51) heraus bewegt wird, so dass die Mischeinrichtung (22, 72) an der Innenseite des Volumenausgleichselements (6, 56) oder der vorderen Innenseite des Behälters (1, 51) anliegt.

23. Verfahren nach einem der Ansprüche 17 bis 21, **dadurch gekennzeichnet, dass** bei dem Durchführen des Verfahrens, die Volumenänderungen in dem Behälter (1, 51) durch das Volumenausgleichselement (4, 6, 56) ausgeglichen werden.

## Claims

1. Device for storing, mixing, and applying polymethylmethacrylate bone cement, comprising a first container (1, 51) for a first pasty component of the bone cement, a dispensing plunger (4, 54) that is arranged such that it can be shifted in the first container (1, 51) for pressing the content of the first container (1, 51) through a dispensing tube (12, 62) situated opposite from the dispensing plunger (4, 54), whereby the dispensing tube (12, 62) is arranged such that it can be rotated and shifted in longitudinal direction through a feed-through in a side of the container (1, 51) opposite from the dispensing plunger (4, 54), and a mixing facility (22, 72) for mixing the content of the first container (1, 51), whereby the mixing facility (22, 72) is arranged in the first container (1, 51) and is secured to the dispensing tube (12, 62), such that the mixing facility (22, 72) can be moved in the first container (1, 51) by moving the dispensing tube (12, 62) in order to mix the content of the first container (1, 51), whereby a closure (20, 71) that can be opened is arranged on the end of the dispensing tube (12, 62) pointing into the inside of the first container (1, 51) and closes the dispensing tube (12, 62), and an axially mobile core (14) is arranged in the dispensing tube (12, 62), and a second container (18) for at least one second component of the bone cement is formed by the space between the closure (20, 71) and the core (14) in the dispensing tube (12, 62), whereby the closure (20, 71) of the second container (18) can be removed from the first end of the dispensing tube (12, 62) through an axial motion of the core (14) and thus the second container can be opened (18) with respect to the first container (1, 51) such that the contents of the second container (18) and of the first container (1, 51) can be mixed with each other in the first container (1, 51), whereby at least one limiting surface of the first container (1, 51) is formed by a mobile volume compensation element (4, 6, 56) and the closure (20, 71) is connected to the dispensing tube (12, 62) by means of a deformable connection (24, 74) such that the closure (20, 71), even when it is open, is connected to the dispensing tube (12, 62) by means of the deformable connection (24, 74), **characterised in that**
a securing element (34, 84) is arranged on the first end of the dispensing tube (12, 62) pointing into the inside and **in that** the securing element (34, 84) can be used to lock the opened closure (20, 71) or to restrict its mobility with respect to the opening of the first end of the dispensing tube (12, 62) such that the opening of the first end of the dispensing tube (12, 62) can no longer be locked or reduced in size by the closure (20, 71).

2. Device according to claim 1, **characterised in that**
the closure (20, 71) is a cap that is plugged onto or arranged on the first end of the dispensing tube (12, 62) pointing into the inside, or the closure (20, 71) is a stopper (20, 71) that is plugged into or arranged in the first end of the dispensing tube (12, 62) pointing into the inside.

3. Device according to claim 1 or 2, **characterised in that**
the deformable connection (24, 74) is a bar (24, 74) that is arched, when the closure (20, 71) closes the dispensing tube (12, 62), whereby the bar (24, 74) and the closure (20, 71) preferably are provided in the form of a single part.

4. Device according to any one of the preceding claims, **characterised in that**
the deformable connection (24, 74) is tensioned and effects a spring force that acts on the closure (20, 71) that moves the closure (20, 71) away from the opening of the first end of the dispensing tube (12, 62) when the closure (20, 71) is detached from the first end of the dispensing tube (12, 62) through the axial motion of the core (14).

5. Device according to any one of the preceding claims, **characterised in that**
the securing element (34, 84) is a securing ring (34, 84) that is plugged onto the first end of the dispensing tube (12, 62) and, in its starting position, projects beyond the first end and can be pushed further onto the first end of the dispensing tube (12, 62), whereby pushing-on the securing ring (34, 84) secures the closure (20, 71) **in that** the new position of the securing ring (34, 84) restricts the deformability of the deformable connection (24, 74).

6. Device according to any one of the preceding claims, **characterised in that**
the dispensing plunger (4, 54) can be or is locked against the first container (1, 51), preferably on the end of the first container (1, 51) opposite from the dispensing tube (12, 62).

7. Device according to any one of the preceding claims, **characterised in that**
the first container (1, 51) comprises a cylindrical internal space and **in that** the dispensing plunger (4, 54) comprises, in the internal space of the first container (1, 51), a fitting shape that corresponds to the footprint of the cylindrical internal space.

8. Device according to any one of the preceding claims, **characterised in that** at least one volume compensation element (4, 6, 56) is arranged in or on the first container (1, 51) such as to be axially mobile.

9. Device according to claim 8, **characterised in that**
the at least one volume compensation element (6, 56) comprises the gas-tight feed-through through which the dispensing tube (12, 62) for operation of the mixing facility (22, 72) is guided.

10. Device according to any one of the preceding claims, **characterised in that** a volume compensation element (4) is implemented through the dispensing plunger (4) and **in that** the motion of the dispensing plunger (4) out of the first container (1) is limited by a limiting element (32), whereby the limiting element (32) preferably is a snap-in mechanism (32) that engages a counter snap-in mechanism on the dispensing plunger (4).

11. Device according to any one of the preceding claims, **characterised in that** a volume compensation element (6, 56) is arranged opposite from the dispensing plunger (4, 54) in the first container (1, 51) as a movably-supported volume compensation element (6, 56), whereby the volume compensation element (6, 56) preferably comprises the gas-tight feed-through for the dispensing tube (12, 62).

12. Device according to any one of the preceding claims, **characterised in that**
the core (14) is provided as closure element of the dispensing opening of the dispensing tube (12, 62), whereby the dispensing opening is arranged opposite from the first opening, whereby the core (14) can be taken out of the dispensing tube (12, 62).

13. Device according to any one of the preceding claims, **characterised in that**
the core (14) comprises, on the side facing the first container (1, 51), a wiper that wipes off powder or cement dough on the inside of the dispensing tube (12, 62) when the core (14) is being pulled out.

14. Device according to any one of the preceding claims, **characterised in that** at least one volume compensation element (6, 56) is supported, such as to be mobile, against the first container (1, 51) by means of an elastic spring (30, 80), whereby the spring (30, 80) pushes the volume compensation element (6, 56) into the internal space of the first container (1, 51).

15. Device according to any one of the preceding claims, **characterised in that**
the side of the container (1, 51) with the dispensing tube (12, 62) is closed by means of a closure cap (26, 76) that comprises a feed-through for the dispensing tube (12, 62) that covers a volume compensation element (6, 56), and **in that** the closure cap (26, 76) comprises at least one opening (28) for facilitating a pressure compensation between the surroundings and the intervening space between the closure cap (26, 76) and the covered volume compensation element (6, 56), whereby it is preferred to have an elastic coil spring (30, 80) arranged between the closure cap (26, 76) and the covered volume compensation element (16, 56) for pushing the volume compensation element (6, 56) into the internal space of the first container (1, 51), whereby the coil spring (30, 80) is particularly preferably arranged about the dispensing tube (12, 62).

16. Device according to any one of the preceding claims, **characterised in that**
the volume compensation element (4, 6, 56) is locked by means of a detachable locking means.

17. Method for producing a polymethylmethacrylate bone cement with a device according to any one of the preceding claims, **characterised by** the steps of: A) Providing the device, whereby the first container (1, 51) is filled with a first liquid or pasty component of the PMMA bone cement and the second container (18) is filled with a second, preferably powdered or pasty, component of the PMMA bone cement; B) opening the second container (18) by advancing the core (14) in the dispensing tube (12, 62) and dispensing the second component from the second container (18) into the first container (1, 51) by further advancing the core (14) in the dispensing tube (12, 62); and C) mixing the two components in the first container (1, 51) by moving the mixing facility (22, 72), whereby moving the mixing facility (22, 72) repeatedly pushes and pulls the dispensing tube (12, 62), which is connected to the mixing facility (22, 72), into and out of the first container (1, 51), whereby the volume change of the content of the first container (1, 51) upon mixing is compensated for by a motion of the at least one volume compensation element (4, 6, 56), whereby the end of the dispensing tube (12, 62) pointing into the inside of the container (1, 51) is pushed all the way onto the dispensing plunger (4, 54), whereby the pressure operates a securing element (34, 84) that is arranged on said end, and whereby the operation of the securing element (34, 84) locks the opened closure (20, 71) or restricts its mobility with respect to the opening of the first end of the dispensing tube (12, 62) such that the opening of the first end of the dispensing tube (12, 62) can no longer be closed or reduced in size by the closure (20, 71).

18. Method according to claim 17, **characterised in that**
the core (14) is removed from the dispensing tube (12, 62) after step B) or step C) and a step D) follows, in which the mixed bone cement is applied by propelling the dispensing plunger (4, 54) in the first container (1, 51).

19. Method according to any one of the claims 17 to 18, **characterised in that**
the mixing facility (22, 72) is connected to the dispensing tube (12, 62) and the content of the first container (1, 51) is mixed by moving the mixing facility (22, 72) in the first container (1, 51) by moving the dispensing tube (12, 62) into and out of the first container (1, 52), whereby, preferably, the mixing facility (22, 72) is additionally rotated by rotating the dispensing tube (12, 62) in the first container (1, 51).

20. Method according to any one of the claims 17 to 19, **characterised in that** preceding step A), the first container (1, 51) is being filled with a component of the bone cement and the inside of the first container (1, 51) is previously being degassed and sterilised.

21. Method according to claim 20, **characterised in that**
the dispensing plunger (4, 54) and/or the volume compensation element (4, 6, 56) is/are being locked for degassing and sterilising the inside of the first container (1, 51).

22. Method according to any one of the claims 17 to 21, **characterised in that**, once the mixing is completed, the dispensing tube (12, 62) is moved in the direction out of the first container (1, 51) such that the mixing facility (22, 72) touches against the inside of the volume compensation element (6, 56) or the front inside of the container (1, 51).

23. Method according to any one of the claims 17 to 21, **characterized in that**
the volume changes in the container (1, 51) are compensated for by the volume compensation element (4, 6 156) during the implementation of the method.

## Revendications

1. Dispositif de stockage, de mélange et d'application de ciment osseux à base de polyméthacrylate de méthyle comportant un premier récipient (1, 51) pour un premier composant pâteux du ciment osseux, un piston d'extraction (4, 54) coulissant dans le premier récipient (1, 51) pour extraire le contenu du premier récipient (1, 51) au travers d'un tube d'extraction (12, 62) situé en face du piston d'extraction (4, 54), le tube d'extraction (12, 62) étant agencé de manière tournante et coulissante dans le sens longitudinal à travers un passage d'un côté du réservoir (1, 51) situé en face du piston d'extraction (4, 54), et un dispositif de mélange (22, 72) pour mélanger le contenu du premier récipient (1, 51), le dispositif de mélange (22, 72) étant agencé dans le premier récipient (1, 51) et fixé au tube d'extraction (12, 62), de sorte que le dispositif de mélange (22, 72) dans le premier récipient (1, 51) soit mobile par le mouvement du tube d'extraction (12, 62) pour mélanger le contenu du premier récipient (1, 51), un obturateur ouvrable (20, 71) fermant le tube d'extraction (12, 62) étant agencé à la première extrémité du tube d'extraction (12, 62) dirigée vers l'intérieur du premier récipient (1, 51), un noyau (14) mobile axialement étant agencé dans le tube d'extraction (12, 62), et, formé par l'espace entre l'obturateur (20,71) et le noyau (14) dans le tube d'extraction (12, 62), un second récipient (18) pour au moins un second composant du ciment osseux, l'obturateur (20, 71) du second récipient (18) étant amovible de la première extrémité du tube d'extraction (12, 62) par un mouvement axial du noyau (14), permettant ainsi d'ouvrir le second récipient (18) vers le premier récipient (1, 51), de sorte que les contenus du premier récipient (1, 51) et du second récipient (18) puissent être mélangés dans le premier récipient (1, 51), au moins une surface de délimitation du premier récipient (1, 51) étant formée par un élément de compensation de volume (4, 6, 56) mobile et l'obturateur (20, 71) étant relié avec le tube d'extraction (12, 62) par l'intermédiaire d'une liaison déformable (24, 74), de sorte que l'obturateur (20, 71) en état ouvert soit aussi relié avec le tube d'extraction (12, 62) par l'intermédiaire de la liaison déformable, **caractérisé en ce, que** soit agencé à la première extrémité du tube d'extraction (12, 62), qui est dirigée vers l'intérieur, un élément de sûreté (34, 84) qui permet de bloquer l'obturateur (20, 71) en état ouvert ou qui permet de limiter son mouvement par rapport à l'ouverture de la première extrémité du tube d'extraction (12, 62), de sorte que l'ouverture de la première extrémité du tube d'extraction (12, 62) ne puisse plus être fermée ou réduite par l'obturateur (20, 71).

2. Dispositif selon la revendication 1, **caractérisé en ce,**
**que** l'obturateur (20,71) soit un capuchon, qui est enfiché ou agencé sur la première extrémité du tube d'extraction (12, 62) dirigée vers l'intérieur, ou que l'obturateur (20,71) soit un bouchon (20, 71), qui est enfiché ou agencé sur la première extrémité du tube d'extraction (12, 62) dirigée vers l'intérieur.

3. Dispositif selon la revendication 1 ou 2, **caractérisé en ce,**
**que** la liaison déformable (24, 74) soit une barrette (24, 74), qui est courbée lorsque l'obturateur (20,71) ferme le tube d'extraction (12, 62), la barrette (24, 74) et l'obturateur (20,71) étant de préférence conçus d'un seul tenant.

4. Dispositif selon l'une des revendications précédentes, **caractérisé en ce,**
**que** la liaison déformable (24, 74) soit tendue et qu'une force de ressort agisse sur l'obturateur (20,71) pour l'éloigner de l'ouverture de la première extrémité du tube d'extraction (12,62), si l'obturateur (20,71) est désolidarisé de la première extrémité du tube d'extraction (12, 62) par le mouvement axial du noyau (14).

5. Dispositif selon l'une des revendications précédentes, **caractérisé en ce,**
**que** l'élément de sûreté (34, 84) soit un anneau de sûreté (34, 84), qui est emmanché sur la première extrémité du tube d'extraction (12, 62), qui dans sa position initiale est en saillie par rapport à la première extrémité et qui peut aussi coulisser sur la première extrémité du tube d'extraction (12, 62), le coulissement de l'anneau de sûreté (34, 84) ayant pour effet de sécuriser l'obturateur (20, 71) par le fait que la possibilité de déformation de la liaison déformable (24, 74) soit restreinte par la nouvelle position de l'anneau de sûreté (34, 84).

6. Dispositif selon l'une des revendications précédentes, **caractérisé en ce,**
**que** le piston d'extraction (4, 54) puisse être bloqué ou est bloqué contre le premier récipient (1, 51), de préférence à l'extrémité du premier récipient (1, 51) située en face du tube d'extraction (12, 62).

7. Dispositif selon l'une des revendications précédentes, **caractérisé en ce,**
**que** le premier récipient (1,51) présente un espace intérieur cylindrique et que le piston d'extraction (4, 54) présente dans l'espace intérieur du premier récipient (1, 51) une forme adaptée à la surface de base de l'espace intérieur cylindrique.

8. Dispositif selon l'une des revendications précédentes, **caractérisé en ce,**
**que** soit agencé dans ou au premier récipient (1, 51) au moins un élément de compensation de volume (4, 6, 56) qui soit mobile axialement.

9. Dispositif selon la revendication 8, **caractérisé en ce,**
**que** l'au moins un élément de compensation de volume (6, 56) comporte le passage étanche aux gaz, à travers lequel passe le tube d'extraction (12, 62) pour la commande du dispositif de mélange (22, 72).

10. Dispositif selon l'une des revendications précédentes, **caractérisé en ce, qu'**un élément de compensation de volume (4) soit réalisé par le piston d'extraction (4) et que le mouvement du piston d'extraction (4) dans le premier récipient (1) soit limité par un élément de limitation (32), l'élément de limitation (32) étant de préférence un crantage (32), qui entre en prise avec un contre-crantage au piston d'extraction (4).

11. Dispositif selon l'une des revendications précédentes, **caractérisé en ce, qu'**un élément de compensation de volume (6, 56) soit agencé dans le premier récipient (1, 51) en face du piston d'extraction (4, 54) en tant qu'élément de compensation de volume (6, 56) mobile, qui comporte de préférence le passage étanche aux gaz pour le tube d'extraction (12, 62).

12. Dispositif selon l'une des revendications précédentes, **caractérisé en ce, que** le noyau (14) soit prévu comme obturateur de l'ouverture d'extraction du tube d'extraction (12, 62), l'ouverture d'extraction étant agencée en face de la première ouverture et le noyau (14) étant amovible du tube d'extraction (12, 62).

13. Dispositif selon l'une des revendications précédentes, **caractérisé en ce, que** le noyau (14) comporte sur sa face dirigées vers le premier récipient (1, 51) un racleur, qui racle de la poudre ou de la pâte de ciment au niveau de la face intérieure du tube d'extraction (12, 62) lorsque le noyau (14) est retiré.

14. Dispositif selon l'une des revendications précédentes, **caractérisé en ce, qu'**au moins un élément de compensation de volume (6, 56) soit monté de manière mobile dans le premier récipient (1, 51) avec un ressort élastique (30, 80), qui pousse l'élément de compensation de volume (6, 56) vers l'espace intérieur du premier récipient (1, 51).

15. Dispositif selon l'une des revendications précédentes, **caractérisé en ce, que** le côté du récipient (1, 51) avec le tube d'extraction (12, 62) soit fermé par un capuchon de fermeture (26, 76), qui comporte un passage pour le tube d'extraction (12, 62), qui couvre un élément de compensation de volume (6, 56) et qui comporte au moins une ouverture (28) pour permettre une compensation de pression entre l'environnement et l'espace intermédiaire entre le capuchon de fermeture (26, 76) et l'élément de compensation de volume (6, 56) couvert, un ressort spiral (30, 80) étant de préférence agencé entre le capuchon de fermeture (26, 76) et l'élément de compensation de volume (6, 56) couvert pour pousser l'élément de compensation de volume (6, 56) dans l'espace intérieur du premier récipient (1, 51), le ressort spiral (30, 80) étant agencé de façon particulièrement préférée autour du tube d'extraction (12, 62).

16. Dispositif selon l'une des revendications précédentes, **caractérisé en ce, que** l'élément de compensation de volume (4, 6, 56) soit bloqué avec un moyen de blocage déblocable.

17. Procédé de fabrication d'un ciment osseux à base de polyméthacrylate de méthyle avec un dispositif selon l'une des revendications précédentes, **caractérisé par** les étapes :
A) Préparation du dispositif, le premier récipient (1, 51) étant rempli d'un premier composant fluide ou pâteux du ciment osseux à base de PMMA et le second récipient (18) étant rempli de préférence d'un second composant pulvérulent ou pâteux du ciment osseux à base de PMMA ;
B) Ouverture du second récipient (18) par l'avancement du noyau (14) dans le tube d'extraction (12, 62) et extraction du second composant du second récipient (18) dans le premier récipient (1, 51) par la poursuite de l'avancement du noyau (14) dans le tube d'extraction (12, 62) ; et
C) Mélange des deux composants dans le premier récipient (1, 51) par le mouvement du dispositif de mélange (22, 72), le tube d'extraction (12, 62) relié au dispositif de mélange (22, 72) étant poussé vers l'avant et tiré vers l'arrière de façon répétée dans le premier récipient (1, 51) lors du mouvement du dispositif de mélange (22, 72), la modification du volume du contenu du premier récipient (1, 51) étant compensée lors du mélange par le mouvement de l'au moins un élément de compensation de volume (4, 6, 56), la première extrémité du tube d'extraction (12, 62) dirigée vers l'intérieur du récipient (1, 51) étant poussée jusqu'au niveau du piston d'extraction (4, 54), un élément de sûreté (34, 84) agencé à cette première extrémité étant actionné par la pression, l'actionnement de l'élément de sûreté (34, 84) ayant pour effet de bloquer l'obturateur (20, 71) ou de limiter sa possibilité de mouvement par rapport à l'ouverture de la première extrémité du tube d'extraction (12, 62), de sorte que l'ouverture de la première extrémité du tube d'extraction (12, 62) ne puisse plus être fermée ou réduite par l'obturateur (20, 71).

18. Procédé selon la revendication 17, **caractérisé en ce,**
**que** l'étape B) ou C) soit suivie par le retrait du noyau (14) du tube d'extraction (12, 62) et qu'ensuite ait lieu une étape D), pendant laquelle le ciment osseux soit appliqué par l'avancement du piston d'extraction (4, 54) dans le premier récipient (1, 51).

19. Procédé selon l'une des revendications 17 à 18, **caractérisé en ce,**
**que** le dispositif de mélange (22, 72) soit lié avec le tube d'extraction (12, 62) et que le contenu du premier récipient (1, 51) soit mélangé par le fait que le dispositif de mélange (22, 72) soit déplacé dans le premier récipient (1, 51) par l'avancement et le recul du tube d'extraction (12, 62) à l'intérieur du premier récipient (1, 51), le dispositif de mélange (22, 72) étant en supplément et de préférence mis en rotation dans le premier récipient (1, 51) par la rotation du tube d'extraction (12, 62).

20. Procédé selon l'une des revendications 17 à 19, **caractérisé en ce, que**, avant l'étape A), le premier récipient (1, 51) soit rempli avec un composant du ciment osseux et que l'intérieur du premier récipient (1, 51) soit préalablement dégazé et stérilisé.

21. Procédé selon la revendication 20, **caractérisé en ce,**
**que** le piston d'extraction (4, 54) et/ou l'élément de compensation de volume (4, 6, 56) soient bloqués pour dégazer et stériliser l'intérieur du premier récipient (1, 51).

22. Procédé selon l'une des revendications 17 à 21, **caractérisé en ce,**
**que**, après l'exécution du mélange, le tube d'extraction (12, 62) soit déplacé en direction de l'extérieur du premier récipient (1, 51), de sorte que le dispositif de mélange (22, 72) soit en appui contre la face intérieure de l'élément de compensation de volume (6, 56) ou contre la face intérieure frontale du récipient (1, 51).

23. Procédé selon l'une des revendications 17 à 21, **caractérisé en ce,**
**que** les modifications des volumes dans le récipient (1, 51) soient compensées par l'élément de compensation de volume (4, 6, 56) pendant l'exécution du procédé.
